# EUROPEAN PATENT SPECIFICATION

(11) **EP 1 670 448 B1**
(45) Date of publication and mention of the grant of the patent: **21.11.2007**
(21) Application number: 04766871.0
(22) Date of filing: 30.09.2004
(51) Int. Cl.: A61K 31/18, A61K 31/426, A61K 31/421, A61P 31/14

(54) **HCV INHIBITING SULFONAMIDES**
HCV-HEMMENDE SULFONAMIDE
SULFONAMIDES INHIBANT LE HCV

(30) Priority: 30.09.2003 EP 03103629; 01.10.2003 US 507535 P
(43) Date of publication of application: 21.06.2006
(73) Proprietor: Tibotec Pharmaceuticals Ltd., Eastgate Little Island Co Cork (IE)
(72) Inventor: SIMMEN, Kenneth Alan, B-3080 Tervuren (BE); VAN ACKER, Koenraad Lodewijk August, B-9140 Temse (BE); WIGERINCK, Piet Tom Bert Paul, B-2840 Terhagen (BE); SURLERAUX, Dominique Louis Nestor Ghislain, B-1440 Braine-le-château (BE); DAMS, Géry Karel Julia, B-3583 Paal-Beringen (BE); QUIRYNEN, Ludo Maria Marcel, B-2320 Hoogstraten (BE); HERTOGS, Kurt, B-2580 Putte (BE); PAUWELS, Rudi Wilfried Jan, CH-1806 Saint-Légier La Chiésaz (CH)
(74) Representative: Daelemans, Frank F.R.
(86) International application number: PCT/EP2004/052388
(87) International publication number: WO 2005/030194

(56) References cited:
- WO-A-95/06030
- WO-A-03/053435
- WO-A-03/064416
- US-A1- 2003 050 229
- US-B1- 6 514 953
- JOHANSSON ANJA ET AL: "Acyl sulfonamides as potent protease inhibitors of the hepatitis C virus full-length NS3 (protease-helicase/NTPase): A comparative study of different C-terminals." BIOORGANIC AND MEDICINAL CHEMISTRY, vol. 11, no. 12, 12 June 2003 (2003-06-12), pages 2551-2568, XP002272318 ISSN: 0968-0896 (ISSN print)
- DATABASE MEDLINE [Online] US NATIONAL LIBRARY OF MEDICINE (NLM), BETHESDA, MD, US; 1 April 2002 (2002-04-01), PALMON RON ET AL: "Lack of hepatotoxicity associated with nonnucleoside reverse transcriptase inhibitors." XP002319202 Database accession no. NLM11917237 & JOURNAL OF ACQUIRED IMMUNE DEFICIENCY SYNDROMES (1999) 1 APR 2002, vol. 29, no. 4, 1 April 2002 (2002-04-01), pages 340-345, ISSN: 1525-4135
- DATABASE MEDLINE [Online] US NATIONAL LIBRARY OF MEDICINE (NLM), BETHESDA, MD, US; September 2003 (2003-09), ROCKSTROH JÜRGEN KURT: "Management of hepatitis B and C in HIV co-infected patients." XP002319203 Database accession no. NLM14562859 & JOURNAL OF ACQUIRED IMMUNE DEFICIENCY SYNDROMES (1999) SEP 2003, vol. 34 Suppl 1, September 2003 (2003-09), pages S59-S65, ISSN: 1525-4135

## Description

The present invention relates to the use of sulfonamides as inhibitors of HCV as well as their use in pharmaceutical compositions aimed to treat or combat HCV infections. The present invention also relates to the use of said sulfonamides in pharmaceutical compositions aimed to treat or combat combined HCV and HIV infections. The present invention also concerns combinations of the present sulfonamides with other anti-HCV agents and/or anti-HIV agents.

Following its discovery in 1989 as the agent implicated in the majority of viral non-A, non-B hepatitis (Choo et al., Science 244, 359-362, 1989), hepatitis C virus (HCV) has become a focus of considerable medical research (Lauer, G.M and Walker, B.D., New Eng. J Med. 345,41-52,2001). HCV is a member of the *Flaviviridae* family of viruses in the *hepacivirus* genus, and is closely related to the *flavivirus* genus, which includes a number of viruses implicated in human disease, such as dengue virus and yellow fever virus, and to the animal *pestivirus* family, which includes bovine viral diarrhea virus (BVDV). HCV is a positive-sense, single-stranded RNA virus, with a genome of around 9,600 bp. The genome comprises both 5' and 3' untranslated regions which adopt RNA secondary structures, and a central open reading frame that encodes a single polyprotein of around 3,010-3,030 amino acids. The polyprotein encodes ten gene products which are generated from the precursor polyprotein by an orchestrated series of co- and posttranslational endoproteolytic cleavages mediated by both host and viral proteases. The viral structural proteins include the core nucleocapsid protein, and two envelope glycoproteins E1 and E2. The non-structural (NS) proteins encode some essential viral enzymatic functions (helicase, polymerase, protease), as well as proteins of unknown function. Replication of the viral genome is mediated by an RNA-dependent RNA polymerase, encoded by non-structural protein 5b (NS5B). In addition to the polymerase, the viral helicase and protease functions, both encoded in the bifunctional NS3 protein, have been shown to be essential for replication of HCV RNA in chimpanzee models of infection (Kolykhalov, A.A., Mihalik, K., Feinstone, S.M., and Rice, C.M. J Virol. 74, 2046-2051, 2000). In addition to the NS3 serine protease, HCV also encodes a metalloproteinase in the NS2 region.

HCV replicates preferentially in hepatocytes but is not directly cytopathic, leading to persistent infection. In particular, the lack of a vigorous T-lymphocyte response and the high propensity of the virus to mutate appear to promote a high rate of chronic infection. There are 6 major HCV genotypes and more than 50 subtypes, which are differently distributed geographically.HCV type 1 is the predominant genotype in the US and Europe. For instance, HCV type 1 accounts for 70 to 75 percent of all HCV infections in the United States. The extensive genetic heterogeneity of HCV has important diagnostic and clinical implications, perhaps explaining difficulties in vaccine development and the lack of response to therapy. An estimated 170 million persons worldwide are infected with hepatitis C virus (HCV). Following the initial acute infection, a majority of infected individuals develop chronic hepatitis, which can progress to liver fibrosis leading to cirrhosis, end-stage liver disease, and HCC (hepatocellular carcinoma)(National Institutes of Health Consensus Development Conference Statement: Management of Hepatitis C. *Hepatology*, 36, 5 Suppl. S3-S20, 2002). Liver cirrhosis due to HCV infection is responsible for about 10,000 deaths per year in the U.S.A. alone, and is the leading cause for liver transplantations. Transmission of HCV can occur through contact with contaminated blood or blood products, for example following blood transfusion or intravenous drug use. The introduction of diagnostic tests used in blood screening has led to a downward trend in post-transfusion HCV incidence. However, given the slow progression to the end-stage liver disease, the existing infections will continue to present a serious medical and economic burden for decades (Kim, W.R. Hepatology, 36, 5 Suppl. S30-S34, 2002).

The treatment of this chronic disease is an unmet clinical need, since current therapy is only partially effective and limited by undesirable side effects.

Current HCV therapies are based on (pegylated) interferon-alpha (IFN-α) in combination with ribavirin. This combination therapy yields a sustained virologic response in more than 40% of patients infected by genotype 1 viruses and about 80% of those infected by genotypes 2 and 3. Beside the limited efficacy on HCV type 1, combination therapy has significant side effects and is poorly tolerated in many patients. For instance, in registration trials of pegylated interferon and ribavirin, significant side effects resulted in discontinuation of treatment in approximately 10 to 14 percent of patients. Major side effects of combination therapy include influenza-like symptoms, hematologic abnormalities, and neuropsychiatric symptoms. The development of more effective, convenient and tolerated treatments is a major public health objective.

Thus, there is a high medical need for low molecular weight HCV inhibitors.

Furthermore, it is known that a large percentage of patients infected with human immunodeficiency virus 1 (HIV) are also infected with HCV, i.e. they are HCV/HIV co-infected. For instance, in the United States, about 25% of persons infected with HIV are also infected with HCV (Sherman et al. 2002 Clin.Infect. Dis. 34:831-837). Given the differences in tissue and cell tropism for HIV and HCV, it seems likely that the impact of HIV on HCV liver disease results from damage to the immune system, rather than from direct effects of HIV enzymes on the HCV replication machinery. The effects of HCV infection on the course of HIV/AIDS are less well defined, and have focused primarily on the impact of liver disease on the tolerability of HAART (highly active antiretroviral therapy). HIV infection appears to adversely affect all stages of HCV infection, leading to increased viral persistence and accelerated progression of HCV-related liver disease. In turn, HCV infection may affect the management of HIV infection, increasing the incidence of liver toxicity caused by antiviral medications. The medical management of HCV in HIV-infected persons remains controversial due to the complexity of both infections, potential drug interactions, and the absence of information (Thomas, D. L. Hepatology 36, 5 Suppl:S201-S209, 2002).

Thus, there is also a high medical need for low molecular weight HCV inhibitors, which may be efficacious in the treatment of HCV in HIV/HCV coinfected patients.

Although HCV/HIV coinfection is common, HCV and HIV belong to two completely different viral orders with different life cycles and host cell requirements. HCV is a flavivirus with a cytoplasmic replication cycle and tropism for the liver, although replication has also been detected in non-liver cells. Flaviviruses have no DNA steps in their life cycle. Although the development of an infectious HCV replication system has proven elusive, it is assumed that HCV may use specific cellular receptors to mediate its predominantly liver-specific replication. Human immunodeficiency viruses type 1 (HIV-1) and type 2 (HIV-2) are human lentiviruses belonging to the Retroviridae family. HIV infects T lymphocytes (particularly CD4 cells) and macrophages. Like other retroviruses HIV exhibits an integration step leading to insertion of the viral DNA sequence into the host DNA. The targeting of CD4+ cells by HIV results from HIV binding to the CD4 cell surface receptor. Further T cell tropism is determined by the utilization of two cellular co-receptors, termed the CC chemokine receptor 5 (CCR5) and the CXC receptor 4 (CXCR4).

Although some of the structures of the present invention have been described in WO 02/083657, WO 02/092595, WO 02/081478, WO 03/53435, PCT/EP03/50057, PCT/EP03/50173 and PCT/EP03/50359 as HIV protease inhibitors, they are not specifically disclosed, suggested or claimed therein in conjunction with HCV infection.

It is therefore surprising that the present sulfonamides are now found to have inhibitory activity against replication of HCV and can therefore be used in pharmaceutical compositions aimed to treat HCV infected patients, even to treat HCV infected patients co-infected with HIV.

### Detailed description

The present invention concerns the use of sulfonamide derivatives having the general formula 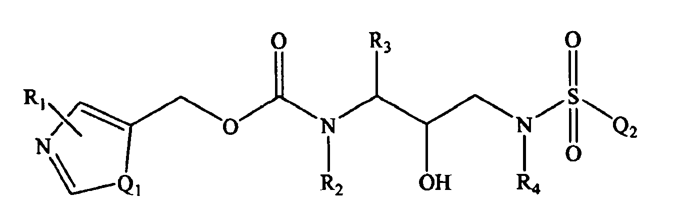 and *N*-oxides, salts, stereoisomeric forms, racemic mixtures, prodrugs and esters thereof, wherein
- Q₁: is -S- or -O-;
- R₁: is hydrogen, C₁₋₆alkyl, hydroxy, amino, halogen, aminoC₁₋₄alkyl and mono-or di(C₁₋₄alkyl)amino;
- R₂, R₁₄ and R₁₅: are, each independently, hydrogen or C₁₋₆alkyl;
- R₃: is C₁₋₆alkyl, aryl, C₃₋₇cycloalkyl, C₃₋₇cycloalkylC₁₋₄alkyl, or arylC₁₋₄alkyl;
- R₄: is hydrogen, C₁₋₄alkyloxycarbonyl, carboxyl, optionally mono- or disubstituted aminocarbonyl, mono- or di(C₁₋₄alkyl)aminocarbonyl, C₃₋₇cycloalkyl, C₂₋₆alkenyl, C₂₋₆alkynyl or C₁₋₆alkyl optionally substituted with one or more substituents each independently selected from aryl, Het¹, Het², C₃₋₇cycloalkyl, C₁₋₄alkyloxy-carbonyl, carboxyl, aminocarbonyl, mono- or di(C₁₋₄alkyl)aminocarbonyl, aminosulfonyl, C₁₋₄alkylS(=O)ₜ, hydroxy, cyano, halogen or amino optionally mono- or di-substituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl;
- Q₂: is a radical of formula (III), (IV), (V), (VI), or (VII) 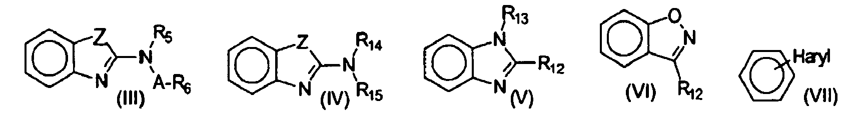 and is be attached to the remainder of the molecule via any available carbon atom of the phenyl or fused phenyl ring,
- Z: is O or S;
- A: is C₁₋₆alkanediyl, -C(=O)-, -C(=S)-, -S(=O)₂-, C₁₋₆alkanediyl-C(=O)-, C₁₋₆alkanediyl-C(=S)- or C₁₋₆alkanediyl-S(=O)₂-; wherein the point of attachment to the nitrogen atom is the C₁₋₆alkanediyl group in those moieties containing said group;
- R₅: is hydrogen, hydroxy, C₁₋₆alkyl, Het¹C₁₋₆alkyl, Het²C₁₋₆alkyl, or aminoC₁₋₆alkyl wherein the amino group may optionally be mono- or di-substituted with C₁₋₄alkyl;
- R₆: is C₁₋₆alkyloxy, Het¹, Het¹oxy, Het², Het²oxy, aryl, aryloxy or amino; and in case -A- is other than C₁₋₆alkanediyl then R₆ may also be C₁₋₆alkyl, Het¹C₁₋₄-alkyl, Het¹oxyC₁₋₄alkyl, Het²C₁₋₄alkyl, Het²oxyC₁₋₄alkyl, arylC₁₋₄alkyl, aryloxyC₁₋₄alkyl or aminoC₁₋₄alkyl; wherein each of the amino groups in the definition of R₆ may optionally be substituted with one or more substituents selected from C₁₋₄alkyl, C₁₋₄alkylcarbonyl, C₁₋₄alkyloxycarbonyl, aryl, arylcarbonyl, aryloxycarbonyl, Het¹, Het², arylC₁₋₄alkyl, Het¹C₁₋₄alkyl or Het²C₁₋₄alkyl;; and
- R₅ and -A-R₆: taken together with the nitrogen atom to which they are attached may also form Het¹ or Het²;
- R₁₂: is hydrogen, -NH₂, -N(R₅)(AR₆), -C₁₋₆alkyl or C₁₋₆alkyl-W-R₁₇, wherein each C₁₋₆alkyl may optionally be substituted with halogen, hydroxy, aryl, Het¹, Het², amino or mono- or di-(C₁₋₄ alkyl)amino;
- W: is oxy, carbonyl, oxycarbonyl, carbonyloxy, oxycarbonyloxy, amino, aminocarbonyl, carbonylamino or sulphur;
- R₁₃: is hydrogen or C₁₋₆-alkyl optionally substituted with a substituent selected from the group consisting of aryl, Het¹, Het², hydroxy, halogen or amino, wherein the amino group may be optionally be mono- or di-substituted with C₁₋₄alkyl;
- R₁₇: is C₁₋₆alkyl, aryl, Het¹ or Het²;
- Haryl: is an aromatic monocyclic, bicyclic or tricyclic heterocycle having 3 to 14 ring members which contains one or more heteroatom ring members selected from nitrogen, oxygen and sulfur and which may optionally be substituted on (i) one or more carbon atoms by a substituent selected from the group consisting of C₁₋₆alkyl, halogen, hydroxy, optionally mono- or di-substituted amino, nitro, cyano, haloC₁₋₆alkyl, carboxyl, C₃₋₇cycloalkyl, optionally mono- or disubstituted aminocarbonyl, methylthio, methylsulfonyl, aryl, -(R₇ₐ)ₙ-M-R_{7b}, Het¹ and Het²; wherein the optional substituents on any amino function in the above group of substituents are independently selected from R₅ and -A-R₆; and on (ii) a nitrogen atom if present by hydroxy or -A-R₆;
- R₇ₐ: is C₁₋₆alkanediyl optionally substituted with one or more substituents selected from, halogen, C₁₋₄alkylcarbonyl, C₁₋₄alkyloxycarbonyl, aryl, arylcarbonyl, aryloxycarbonyl, Het¹ or Het²;
- R_{7b}: is C₁₋₆alkyl optionally substituted with one or more substituents selected from halogen, C₁₋₄alkylcarbonyl, C₁₋₄alkyloxycarbonyl, aryl, arylcarbonyl, aryloxycarbonyl, Het¹ or Het²;
- R₈: is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het² or Het²C₁₋₆alkyl;
- M: is defined by -C(=O)-, -O-C(=O)-, -C(=O)-O-, -CH₂-CHOH-, -CHOH-CH₂-, -NR₈-C(=O)-, -(C=O)-NR₈-, -S(=O)₂-, -O-, -S-, -O-S(=O)₂-, -S(=O)₂-O-, -NR₈-S(=O)₂ or -S(=O)₂-NR₈-;
- n: is zero or 1;
for the manufacture of a medicament useful for inhibiting HCV activity in a mammal infected with HCV.

The further embodiments will become apparent throughout the description. It will be appreciated that the compounds described herein are intended for the use according to the invention.

This invention also envisions the quaternization of the nitrogen atoms of the present compounds. A basic nitrogen can be quaternized with any agent known to those of ordinary skill in the art including, for instance, lower alkyl halides, dialkyl sulfates, long chain halides and aralkyl halides.

Whenever the term "substituted" is used in defining the compounds of formula (I), it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

As used herein, the term "halo" or "halogen" as a group or part of a group is generic for fluoro, chloro, bromo or iodo.

The term "C₁₋₄alkyl" as a group or part of a group defines straight and branched chained saturated hydrocarbon radicals having from 1 to 4 carbon atoms, such as, for example, methyl, ethyl, propyl, butyl and 2-methyl-propyl, and the like.

The term "C₁₋₆alkyl" as a group or part of a group defines straight and branched chained saturated hydrocarbon radicals having from 1 to 6 carbon atoms such as the groups defined for C₁₋₄alkyl and pentyl, hexyl, 2-methylbutyl, 3-methylpentyl and the like.

The term "C₁₋₆alkanediyl" as a group or part of a group defines bivalent straight and branched chained saturated hydrocarbon radicals having from 1 to 6 carbon atoms such as, for example, methylene, ethan-1,2-diyl, propan-1,3-diyl, propan-1,2-diyl, butan-1,4-diyl, pentan-1,5-diyl, hexan-1,6-diyl, 2-methylbutan-1,4-diyl, 3-methylpentan-1,5-diyl and the like.

The term "C₂₋₆alkenyl" as a group or part of a group defines straight and branched chained hydrocarbon radicals having from 2 to 6 carbon atoms containing at least one double bond such as, for example, ethenyl, propenyl, butenyl, pentenyl, hexenyl and the like.

The term "C₂₋₆alkynyl" as a group or part of a group defines straight and branched chained hydrocarbon radicals having from 2 to 6 carbon atoms containing at least one triple bond such as, for example, ethynyl, propynyl, butynyl, pentynyl, hexynyl and the like.

The term "C₃₋₇cycloalkyl" as a group or part of a group is generic to cyclopropyl, cyclobutyl, cyclopentyl, cyclohexyl or cycloheptyl.

The term "aryl" as a group or part of a group is meant to include phenyl and naphtyl, which both may be optionally substituted with one or more substituents independently selected from the group consisting of C₁₋₆alkyl, C₁₋₆alkyloxy, halogen, hydroxy, optionally mono- or disubstituted amino, nitro, cyano, haloC₁₋₆alkyl, carboxyl, C₁₋₆alkoxycarbonyl, C₃₋₇cycloalkyl, Het¹, optionally mono- or disubstituted aminocarbonyl, optionally mono- or disubstituted aminoC₁₋₆alkyl, methylthio, methylsulfonyl, and phenyl optionally substituted with one or more substituents selected from the group consisting of C₁₋₆alkyl, halogen, C₁₋₆alkyloxy, hydroxy, optionally mono- or disubstituted amino, nitro, cyano, carboxyl, haloC₁₋₆alkyl, C₁₋₆alkoxycarbonyl, C₃₋₇cycloalkyl, Het¹, optionally mono- or di-substituted aminocarbonyl, methylthio and methylsulfonyl; wherein the optional substituents on any amino function of the above two groups of substituents are independently selected from the group consisting of C₁₋₆alkylcarbonyl, C₁₋₆alkyl, C₁₋₆alkyloxy-A-, Het¹-A-, Het¹C₁₋₆alkyl, Het¹C₁₋₆alkyl-A-, Het¹oxy-A-, Het¹oxyC₁₋₄akyl-A-, phenyl-A-, phenyl-oxy-A-, phenyloxyC₁₋₄alkyl-A-, phenyl-, C₁₋₆alkyl-A-, C₁₋₆alkyloxycarbonylamino-A-, amino-A-, aminoC₁₋₆alkyl and amino- C₁₋₆alkyl-A-, wherein A is as defined above and wherein each of the amino groups in the latter group of substituents may optionally be mono- or, where possible, di-substituted with C₁₋₄alkyl.

The term "haloC₁₋₆alkyl" as a group or part of a group is defined as C₁₋₆alkyl substituted with one or more halogen atoms, preferably, chloro or fluoro atoms, more preferably fluoro atoms. Preferred haloC₁₋₆alkyl groups include for instance trifluoromethyl and difluoromethyl.

The term "Het¹" as a group or part of a group is defined as a saturated or partially unsaturated monocyclic, bicyclic or tricyclic heterocycle having preferably 3 to 14 ring members, more preferably 5 to 10 ring members and more preferably 5 to 8 ring members, which contains one or more heteroatom ring members selected from nitrogen, oxygen or sulfur and which is optionally substituted on one or more carbon atoms by a substituent selected from the group consisting of C₁₋₆alkyl, C₁₋₆alkyloxy, halogen, hydroxy, oxo, optionally mono- or disubstituted amino, nitro, cyano, haloC₁₋₆alkyl, carboxyl, C₁₋₆alkoxycarbonyl, C₃₋₇cycloalkyl, optionally mono- or disubstituted aminocarbonyl, optionally mono- or disubstituted aminoC₁₋₆alkyl, methylthio, methylsulfonyl, phenyl and a saturated or partially unsaturated monocyclic, bicyclic or tricyclic heterocycle having 3 to 14 ring members which contains one or more heteroatom ring members selected from nitrogen, oxygen or sulfur; and wherein the optional substituents on any amino function in the above mentioned group of substituents are independently selected from the group of substituents consisting of C₁₋₆alkyl, C₁₋₆alkylcarbonyl, C₁₋₆alkyloxy-A-, Het²-A-, Het²C₁₋₆alkyl, Het²C₁₋₆alkyl-A-, Het²oxy-A-, Het²oxyC₁₋₄akyl-A-, phenyl-A-, phenyloxy-A-, phenyloxyC₁₋₄alkyl-A-, phenylC₁₋₆alkyl-A-, C₁₋₆alkyloxycarbonylamino-A-, amino-A-, aminoC₁₋₆alkyl and aminoC₁₋₆alkyl-A-; wherein A is as defined above and wherein each of the amino groups in the latter group of substituents may optionally be mono- or where possible di-substituted with C₁₋₄alkyl.

The term "Het²" as a group or part of a group is defined as an aromatic monocyclic, bicyclic or tricyclic heterocycle having preferably 3 to 14 ring members, more preferably 5 to 10 ring members and more preferably 5 to 6 ring members, which contains one or more heteroatom ring members selected from nitrogen, oxygen or sulfur and which is optionally substituted on one or more carbon atoms by a substituent selected from the group of substituents consisting of C₁₋₆alkyl, C₁₋₆alkyloxy, halogen, hydroxy, optionally mono- or disubstituted amino, nitro, cyano, haloC₁₋₆alkyl, carboxyl, C₁₋₆alkoxycarbonyl, C₃₋₇cycloalkyl, optionally mono- or disubstituted aminocarbonyl, optionally mono- or disubstituted aminoC₁₋₆alkyl, methylthio, methylsulfonyl, aryl, and an saturated, partially saturated and aromatic monocyclic, bicyclic or tricyclic heterocycle having 3 to 14 ring members; wherein the optional substituents on any amino function in the above group of substituents are independently selected from the group of substituents consisting of C₁₋₆alkyl, C₁₋₆alkylcarbonyl, C₁₋₆alkyloxy-A-, C₁₋₄akyl-A-, aryl-A-, aryloxy-A-, aryloxyC₁₋₄alkyl-A-, arylC₁₋₆alkyl-A-, C₁₋₆alkyloxycarbonylamino-A-, amino-A-, aminoC₁₋₆alkyl and aminoC₁₋₆alkyl-A-; wherein A is as defined above and wherein each of the amino groups in the latter group of substituents may optionally be mono- or where possible di-substituted with C₁₋₄alkyl.

As used herein, the term (=O) forms a carbonyl moiety with the carbon atom to which it is attached.

As used herein before, the term "one or more" covers the possibility of all the available C-atoms, where appropriate, to be substituted, preferably, one, two or three. Similarly, the term "one or more" covers the possibility of all the available N-atoms, where applicable, to be substituted, preferably, one or two.

When any variable (e.g. halogen or C₁₋₄alkyl) occurs more than one time in any constituent, each definition is independent.

The term "prodrug" as used throughout this text means the pharmacologically acceptable derivatives such as esters, amides and phosphates, such that the resulting in vivo biotransformation product of the derivative is the active drug as defined in the compounds of formula (I). The reference by Goodman and Gilman (The Pharmacological Basis of Therapeutics, 8th ed, McGraw-Hill, Int. Ed. 1992, "Biotransformation of Drugs", p 13-15) describes prodrugs generally. Prodrugs of a compound of the present invention are prepared by modifying functional groups present in the compound in such a way that the modifications are cleaved, either in routine manipulation or in vivo, to the parent compound. Prodrugs include compounds of the present invention wherein a hydroxy group, for instance the hydroxy group on the asymmetric carbon atom, or an amino group is bonded to any group that, when the prodrug is administered to a patient, cleaves to form a free hydroxyl or free amino, respectively.

Typical examples of prodrugs are described for instance in WO 99/33795, WO 99/33815, WO 99/33793 and WO 99/33792.

Prodrugs are characterized by excellent aqueous solubility, increased bioavailability and are readily metabolized into the active inhibitors *in vivo.*

For therapeutic use, the salts of the compounds of formula (I) are those wherein the counterion is pharmaceutically or physiologically acceptable. However, salts having a pharmaceutically unacceptable counterion may also find use, for example, in the preparation or purification of a pharmaceutically acceptable compound of formula (I).

All salts, whether pharmaceutically acceptable or not are included within the ambit of the present invention.

The pharmaceutically acceptable or physiologically tolerable addition salt forms which the compounds of the present invention are able to form can conveniently be prepared using the appropriate acids, such as, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, *p*-toluenesulfonic, cyclamic, salicylic, *p*-amino-salicylic, pamoic and the like acids.

Conversely said acid addition salt forms can be converted by treatment with an appropriate base into the free base form.

The compounds of formula (I) containing an acidic proton may also be converted into their non-toxic metal or amine addition salt form by treatment with appropriate organic and inorganic bases. Appropriate base salt forms comprise, for instance, the ammonium salts, the alkali and earth alkaline metal salts, e.g. the lithium, sodium, potassium, magnesium, calcium salts and the like, salts with organic bases, e.g. the benzathine, N-methyl, -D-glucamine, hydrabamine salts, and salts with amino acids such as, for example, arginine, lysine and the like.

Conversely said base addition salt forms can be converted by treatment with an appropriate acid into the free acid form.

The term "salts" also comprises the hydrates and the solvent addition forms which the compounds of the present invention are able to form. Examples of such forms are e.g. hydrates, alcoholates and the like.

The *N*-oxide forms of the present compounds are meant to comprise the compounds of formula (I) wherein one or several nitrogen atoms are oxidized to the so-called *N*-oxide.

The present compounds may also exist in their tautomeric forms. Such forms, although not explicitly indicated in the above formula are intended to be included within the scope of the present invention.

The term stereochemically isomeric forms of compounds of the present invention, as used hereinbefore, defines all possible compounds made up of the same atoms bonded by the same sequence of bonds but having different three-dimensional structures which are not interchangeable, which the compounds of the present invention may possess. Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound All stereochemically isomeric forms of the compounds of the present invention both in pure form or in admixture with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term 'stereoisomerically pure' concerns compounds or intermediates having a stereoisomeric excess of at least 80% (i. e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms 'enantiomerically pure' and 'diastereomerically pure' should be understood in a similar way, but then having regard to the enantiomeric excess, respectively the diastereomeric excess of the mixture in question.

Pure stereoisomeric forms of the compounds and intermediates of this invention may be obtained by the application of art-known procedures. For instance, enantiomers may be separated from each other by the selective crystallization of their diastereomeric salts with optically active acids. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound will be synthesized by stereospecific methods of preparation. These methods will advantageously employ enantiomerically pure starting materials.

The diastereomeric racemates of formula (I) can be obtained separately by conventional methods. Appropriate physical separation methods which may advantageously be employed are, for example, selective crystallization and chromatography, e.g. column chromatography.

It is clear to a person skilled in the art that the compounds of formula (I) contain at least one asymmetric center and thus may exist as different stereoisomeric forms. The absolute configuration of each asymmetric center that may be present in the compounds of formula (I) may be indicated by the stereochemical descriptors R and S, this R and S notation corresponding to the rules described in Pure Appl. Chem. 1976, 45, 11-30. The carbon atom marked with the asterisk (*) preferably has the R configuration.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

Whenever used hereinafter, the term "compounds of formula (I)", or "the present compounds" or similar term is meant to include the compounds of general formula (I), their *N*-oxides, salts, stereoisomeric forms, racemic mixtures, prodrugs and esters, as well as their quaternized nitrogen analogues.

A particular group of compounds are those compounds of formula (I) wherein one or more of the following restrictions apply :
- R₁: is hydrogen or halogen;
- R₂: is hydrogen;
- R₃: is arylC₁₋₄alkyl;
- R₄: C₁₋₆alkyl;
- Q₂: is a radical of formula (III) wherein Z is O;
- Q₂: is a radical of formula (III) wherein Z is S;
- Q₂: is a radical of formula (IV) wherein Z is O;
- Q₂: is a radical of formula (IV) wherein Z is S;
- Q₂: is a radical of formula (V);
- Q₂: is a radical of formula (VI);
- Q₂: is a radical of formula (VII);
- R₅: is hydrogen or C₁₋₆alkyl; or taken together with -A-R₆ and with the nitrogen atom to which it is attached forms a Het¹;
- A: is C₁₋₆alkanediyl or -C(=O)-;
- R₆: is C₁₋₆alkyloxy, Het¹, Het², aryl or amino; and in case -A- is other than C₁₋₆alkanediyl then R₆ may also be C₁₋₆alkyl, Het¹C₁₋₄-alkyl, Het²C₁₋₄alkyl, arylC₁₋₄alkyl or aminoC₁₋₄alkyl; wherein each of the amino groups in the definition of R₆ may optionally be substituted with one or more substituents selected from C₁₋₄alkyl, arylC₁₋₄alkyl, Het¹C₁₋₄alkyl or Het²C₁₋₄alkyl;
- R₁₂: is hydrogen;
- R₁₃: is hydrogen or C₁₋₆-alkyl optionally substituted with aryl;
- Haryl: is thiazolyl or oxazolyl which may both optionally be substituted with C₁₋₆alkyl or Het²amino;

A special group of compounds are those compounds of formula (I) wherein R₂ is hydrogen.

Another featured group of compounds are those compounds of formula (I), wherein R₃ is arylC₁₋₄alkyl and R₂ is hydrogen.

Another featured group of compounds are those compounds of formula (I), wherein R₄ is C₁₋₆alkyl, and in particular wherein R₄ is C₁₋₆alkyl, R₂ is hydrogen, R₃ is arylC₁₋₄alkyl.

Another featured group of compounds are those compounds of formula (I), as defined herein wherein R₄ is tert-butyl, butyl or isobutyl.

Another featured group of compounds are those compounds of formula (I), as defined herein, wherein Q₂ is a radical of formula (III), and in particular, those compounds of formula (I), as defined herein, wherein Q₂ is a radical of formula (III), R₄ is C₁₋₆alkyl, R₂ is hydrogen, R₃ is arylC₁₋₄alkyl.

Another featured group of compounds are those compounds of formula (I) as defined herein, wherein Q₂ is a radical of formula (IV), and in particular those compounds of formula (I), as defined herein, wherein Q₂ is a radical of formula (IV), R₄ is C₁₋₆alkyl, R₂ is hydrogen, R₃ is arylC₁₋₄alkyl.

Another featured group of compounds are those compounds of formula (I) as defined herein, wherein Q₂ is a radical of formula (V), and in particular those compounds of formula (I), as defined herein, wherein Q₂ is a radical of formula (V), R₄ is C₁₋₆alkyl, R₂ is hydrogen, R₃ is arylC₁₋₄alkyl.

Another featured group of compounds are those compounds of formula (I) as defined herein, wherein Q₂ is a radical of formula (VI), and in particular those compounds of formula (I), as defined herein, wherein Q₂ is a radical of formula (VI), R₄ is C₁₋₆alkyl, R₂ is hydrogen, R₃ is arylC₁₋₄alkyl.

Another featured goup of compounds are those compounds of formula (I) as defined herein, wherein Q₂ is a radical of formula (VII), and in particular those compounds of formula (I), as defined herein, wherein Q₂ is a radical of formula (VII), R₄ is C₁₋₆alkyl, R₂ is hydrogen, R₃ is arylC₁₋₄alkyl.

Another featured group of compounds are those compounds of formula (I), wherein Q₂ is a radical of formula (III), and wherein A is -C(=O)- or C₁₋₆alkanediyl, R₅ is hydrogen or C₁₋₆alkyl; or taken together with -A-R₆ and with the nitrogen atom to which it is attached forms a Het¹; R₆ is C₁₋₆alkyloxy, Het¹, Het², aryl or amino; and in case -A- is other than C₁₋₆alkanediyl then R₆ may also be C₁₋₆alkyl, Het¹C₁₋₄-alkyl, Het²C₁₋₄alkyl, arylC₁₋₄alkyl or aminoC₁₋₄alkyl; wherein each of the amino groups in the definition of R₆ may optionally be substituted with one or more substituents selected from C₁₋₄alkyl, arylC₁₋₄alkyl, Het¹C₁₋₄alkyl or Het²C₁₋₄alkyl.

Another interesting group of compounds are those compounds of formula (I) as defined herein, wherein Q₂ is a radical of formula (VII) and wherein R₄ is C₁₋₆alkyl, R₂ is hydrogen, R₃ is arylC₁₋₄alkyl and the Haryl moiety is selected from thiazolyl, imidazolyl, oxazolyl, oxadiazolyl, pyrazolyl, pyrazinyl, imidazolinonyl, quinolinyl, isoquinolinyl, indolyl, pyridazinyl, pyridinyl, pyrrolyl, pyranyl, pyrimidinyl, furanyl, triazolyl, tetrazolyl, benzofuranyl, benzoxazolyl, isoxazolyl, isothiazolyl, thiadiazolyl, thiophenyl, tetrahydrofurofuranyl, tetra-hydropyranofuranyl, benzothiophenyl, carbazolyl, imidazolonyl, oxazolonyl, indolizinyl, triazinyl or quinoxalinyl, and (i) which is optionally substituted on one or more carbon atoms by halogen, optionally mono- or disubstituted amino, nitro, cyano, C₃₋₇cycloalkyl, optionally mono- or disubstituted aminocarbonyl, -(R₇ₐ)ₙ-M-R_{7b}, Het¹ or Het²; wherein the optional substituents on any amino function are independently selected from R₅ and -A-R₆.

Accordingly, the present invention relates particularly to the use as defined herein of the sulfonamide selected from the following group:
{3-[(2-Acetylamino-benzooxazole-6-sulfonyl)-isobutyl-amino]-1-benzyl-2-hydroxypropyl}-carbamic acid thiazol-5-ylmethyl ester;
[1-Benzyl-2-hydroxy-3-({2-[(6-hydroxy-pyridine-3-carbonyl)-amino]-benzooxazole-6-sulfonyl}-isobutyl-amino)-propyl]-carbamic acid thiazol-5-ylmethyl ester;
[1-Benzyl-2-hydroxy-3-(isobutyl-{2-[(pyridine-3-carbonyl)-amino]-benzooxazole-6-sulfonyl}-amino)-propyl]-carbamic acid thiazol-5-ylmethyl ester;
{1-Benzyl-2-hydroxy-3-[isobutyl-(2-pyrrolidin-1-yl-benzooxazole-6-sulfonyl)-amino]-propyl}-carbamic acid thiazol-5-ylmethyl ester;
[1-Benzyl-2-hydroxy-3-(isobutyl-{2-[2-(4-methyl-piperazin-1-yl)-acetylamino]-benzooxazole-6-sulfonyl}-amino)-propyl]-carbamic acid thiazol-5-ylmethyl ester;
[1-Benzyl-3-({2-[(furan-3-carbonyl)-methyl-amino]-benzooxazole-6-sulfonyl}-isobutyl-amino)-2-hydroxy-propyl]-carbamic acid thiazol-5-ylmethyl ester;
[1-Benzyl-2-hydroxy-3-(isobutyl-{2-[(1-methyl-pyrrolidine-2-carbonyl)-amino]-benzooxazole-6-sulfonyl}-amino)-propyl]-carbamic acid thiazol-5-ylmethyl ester;
{1-Benzyl-3-[(3-benzyl-3H-benzoimidazole-5-sulfonyl)-isobutyl-amino]-2-hydroxypropyl}-carbamic acid thiazol-5-ylmethyl ester;
{3-[(2-Amino-benzothiazole-6-sulfonyl)-isobutyl-amino]-1-benzyl-2-hydroxy-propyl}-carbamic acid thiazol-5-ylmethyl ester;
(1-Benzyl-3-{[2-(2-dimethylamino-ethylamino)-benzothiazole-6-sulfonyl]-isobutyl-amino}-2-hydroxy-propyl)-carbamic acid thiazol-5-ylmethyl ester;
(1-Benzyl-2-hydroxy-3-{isobutyl-[2-(2-pyrrolidin-1-yl-ethylamino)-benzothiazole-6-sulfonyl]-amino}-propyl)-carbamic acid thiazol-5-ylmethyl ester;
(1-Benzyl-2-hydroxy-3-{isobutyl-[2-(2-pyrrolidin-1-yl-ethylamino)-benzothiazole-6-sulfonyl]-amino}-propyl)-carbamic acid thiazol-5-ylmethyl ester;
(1-Benzyl-2-hydroxy-3-{isobutyl-[2-(2-piperazin-1-yl-ethylamino)-benzothiazole-6-sulfonyl]-amino}-propyl)-carbamic acid thiazol-5-ylmethyl ester;
{3-[(2-Amino-benzooxazole-6-sulfonyl)-isobutyl-amino]-1-benzyl-2-hydroxy-propyl}-carbamic acid thiazol-5-ylmethyl ester;
[1-Benzyl-2-hydroxy-3-(isobutyl-{2-[(5-oxo-pyrrolidine-2-carbonyl)-amino]-benzooxazole-6-sulfonyl}-amino)-propyl]-carbamic acid thiazol-5-yl methyl ester;
[1-Benzyl-3-({2-[(furan-3-carbonyl)-amino]-benzooxazole-6-sulfonyl}-isobutyl-amino)-2-hydroxy-propyl]-carbamic acid thiazol-5-ylmethyl ester;
[1-Benzyl-2-hydroxy-3-(isobutyl-{2-[(pyridine-2-carbonyl)-amino]-benzooxazole-6-sulfonyl}-amino)-propyl]-carbamic acid thiazol-5-ylmethyl ester;
{3-[(2-Amino-benzooxazole-6-sulfonyl)-isobutyl-amino]-1-benzyl-2-hydroxy-propyl}-carbamic acid 2-chloro-thiazol-5-ylmethyl ester;
(1-Benzyl-3-{[2-(2-dimethylamino-acetylamino)-benzooxazole-6-sulfonyl]-isobutyl-amino}-2-hydroxy-propyl)-carbamic acid thiazol-5-ylmethyl ester;
{1-Benzyl-2-hydroxy-3-[isobutyl-(2-piperazin-1-yl-benzooxazole-6-sulfonyl)-amino]-propyl}-carbamic acid thiazol-5-ylmethyl ester;
{1-Benzyl-2-hydroxy-3-[isobutyl-(2-piperidin-1-yl-benzooxazole-6-sulfonyl)-amino]-propyl}-carbamic acid thiazol-5-ylmethyl ester;
[1-Benzyl-2-hydroxy-3-(isobutyl-{2-[(pyridine-4-carbonyl)-amino]-benzooxazole-6-sulfonyl}-amino)-propyl]-carbamic acid thiazol-5-ylmethyl ester;
{1-Benzyl-2-hydroxy-3-[isobutyl-(3-isobutyl-3H-benzoimidazole-5-sulfonyl)-amino]-propyl}-carbamic acid thiazol-5-ylmethyl ester;
[1-Benzyl-2-hydroxy-3-(isobutyl-{4-[2-(pyridin-4-ylamino)-thiazol-4-yl]-benzenesulfonyl}-amino)-propyl]-carbamic acid thiazol-5-ylmethyl ester;
{3-[(2-Amino-benzooxazole-6-sulfonyl)-isobutyl-amino]-1-benzyl-2-hydroxy-propyl}-carbamic acid thiazol-5-ylmethyl ester;
an *N*-oxide, a salt or stereoisomeric form thereof.

Interestingly, the compounds of the present invention may comprise chemically reactive moieties or groups capable of forming covalent bonds to localized sites such that said compound have increased tissue retention and half-lives. The term "chemically reactive group" as used herein refers to chemical groups capable of forming a covalent bond. Reactive groups will generally be stable in an aqueous environment and will usually be carboxy, phosphoryl, or convenient acyl group, either as an ester or a mixed anhydride, or an imidate, or a maleimidate thereby capable of forming a covalent bond with functionalities such as an amino group, a hydroxy or a thiol at the target site on for example blood components.

Upon administration to an individual in need thereof, said compound is capable of forming covalent bonds to localized sites, with blood component for example, such that said compound according to the invention has increased tissue retention and half-lives. Usually, the covalent bond that is formed should be able to be maintained during the lifetime of the blood component, unless it is intended to be a release site. A major advantage of said new compound is the small amount of compound necessary to provide an effective effect. The reasons for this advantage are explained by the targeting of the delivery, the high yield of reaction between the reactive entity Y and reactive functionality and the irreversible nature of the bond formed after reaction. Furthermore, once bound to the membrane or tissue said compound according to the invention is not susceptible to liver metabolism, kidney filtration and excretion, and may even be protected from protease (inclusive of endopeptidase) activity which usually leads to loss of activity and accelerated elimination.

"Blood components" as used herein refers to either fixed or mobile blood components. Fixed blood components are non-mobile blood components and include tissues, membrane receptors, interstitial proteins, fibrin proteins, collagens, platelets, endothelial cells, epithelial cells and their associated membrane and membranous receptors, somatic body cells, skeletal and smooth muscle cells, neuronal components, osteocytes and osteoclasts and all body tissues especially those associated with the circulatory and lymphatic systems. Mobile blood components are blood components that do not have a fixed situs for any extended period of time, generally not exceeding 5, more usually one minute. These blood components are not membrane-associated and are present in the blood for extended periods of time and are present in a minimum concentration of at least 0.1 µg/ml. Mobile blood components include serum albumin, transferrin, ferritin and immunoglobulins such as IgM and IgG. The half-life of mobile blood components is at least about 12 hours.

The compounds of formula (I) can generally be prepared using procedures analogous to those procedures described in WO 95/06030, WO 96/22287, WO 96/28418, WO 96/28463, WO 96/28464, WO 96/28465 or WO 97/18205.

Particular reaction procedures to make the present compounds are specifically described in WO 02/083657, WO 02/092595, WO 02/081478, WO 03/53435, PCT/EP03/50057, PCT/EP03/50173 or PCT/EP03/50359. In these preparations, the reaction products may be isolated from the medium and, if necessary, further purified according to methodologies generally known in the art such as, for example, extraction, crystallization, trituration and chromatography.

It will be appreciated that the intermediates, wherein -A-R₆ is hydrogen, may also have pharmalogical properties, similar to those pharmalogical properties of the compounds of formula (I). Using methods known to the person skilled in the art, such as for instance described below, he will be able to test the efficacy and efficiency of these intermediates as well as the compounds described herein, without undue burden or the application of inventive skill.

The present compounds can thus be used in animals, preferably in mammals, and in particular in humans as pharmaceuticals per se, in mixtures with one another or in the form of pharmaceutical preparations.

Furthermore, the present invention relates to pharmaceutical preparations which as active constituents contain an effective dose of at least one of the compounds of formula (I) in addition to customary pharmaceutically innocuous excipients and auxiliaries. The pharmaceutical preparations normally contain 0.1 to 90% by weight of a compound of formula (I). The pharmaceutical preparations can be prepared in a manner known per se to one of skill in the art. For this purpose, at least one of a compound of formula (I), together with one or more solid or liquid pharmaceutical excipients and/or auxiliaries and, if desired, in combination with other pharmaceutical active compounds, are brought into a suitable administration form or dosage form which can then be used as a pharmaceutical in human medicine or veterinary medicine.

Pharmaceuticals which contain a compound according to the invention can be administered orally, parenterally, e.g., intravenously, rectally, by inhalation, or topically, the preferred administration being dependent on the individual case, e.g., the particular course of the disorder to be treated. Oral administration is preferred.

The person skilled in the art is familiar on the basis of his expert knowledge with the auxiliaries which are suitable for the desired pharmaceutical formulation. Beside solvents, gel-forming agents, suppository bases, tablet auxiliaries and other active compound carriers, antioxidants, dispersants, emulsifiers, antifoams, flavor corrigents, preservatives, solubilizers, agents for achieving a depot effect, buffer substances or colorants are also useful.

Due to their favorable antiviral properties, as will be apparent from the examples, the compounds of the present invention are useful in the treatment of individuals infected by HCV and for the prophylaxis of these individuals. In general, the compounds of the present invention may be useful in the treatment of warm-blooded animals infected with flaviviruses. Conditions which may be prevented or treated with the compounds of the present invention, especially conditions associated with HCV and other pathogenic flaviviruses, such as Yellow fever, Dengue fever (types 1-4), St. Louis encephalitis, Japanese encephalitis, Murray valley encephalitis, West Nile virus and Kunjin virus. The conditions associated with HCV include progressive liver fibrosis, inflammation and necrosis leading to cirrhosis, end-stage liver disease, and HCC; and for the other pathogenic flaviruses the conditions include yellow fever, dengue fever, haemorraghic fever and encephalitis.

The compounds of the present invention or any subgroup thereof may therefore be used as medicines against the above-mentioned conditions. Said use as a medicine or method of treatment comprises the systemic administration to HCV-infected subjects of an amount effective to combat the conditions associated with HCV and other pathogenic flaviviruses. Consequently, the compounds of the present invention can be used in the manufacture of a medicament useful for treating conditions associated with HCV and other pathogenic flaviviruses, in particular medicaments useful for treating patients co-infected with HCV and HIV virus.

In an embodiment, the invention relates to the use of a compound of formula (I) or any subgroup thereof as defined herein in the manufacture of a medicament for treating or combating infection or disease associated with HCV infection in a mammal. The invention also relates to a method of treating a flaviviral infection, or a disease associated with flavivirus infection comprising administering to a mammal in need thereof an effective amount of a compound of formula (I) or a subgroup thereof as defined herein.

In another embodiment, the present invention relates to the use of formula (I) or any subgroup thereof as defined herein for the manufacture of a medicament useful for inhibiting HCV activity in a mammal infected with flaviviruses, in particular HCV.

In another embodiment, the present invention relates to the use of formula (I) or any subgroup thereof as defined herein for the manufacture of a medicament useful for inhibiting HCV activity in a mammal infected with flaviviruses, wherein said HCV is inhibited in its replication.

Also, the combination of previously known anti-HCV compound, such as, for instance, interferon-α (IFN-α), pegylated interferon-α and/or ribavirin, and a compound of the present invention can be used as a medicine in a combination therapy. The term "combination therapy" relates to a product containing mandatory (a) a compound of the present invention, and (b) optionally another anti-HCV compound, and/or possibly (c) an anti-HIV compound, as a combined preparation for simultaneous, separate or sequential use in treatment of HCV infections, in particular, in the treatment of infections with HCV type 1, and possibly coinfected with HIV. Thus, to combat or treat HCV infections, or the infection and disease associated with HCV infections, such as liver fibrosis, inflammation, necrosis leading to cirrhosis, end-stage liver disease and HCC, the compounds of this invention may be co-administered in combination with for instance, interferon-α (IFN-α), pegylated interferon-α and/or ribavirin, as well as therapeutics based on antibodies targeted against HCV epitopes, small interfering RNA (Si RNA), ribozymes, DNAzymes, antisense RNA, small molecule antagonists of for instance NS3 protease, NS3 helicase and NS5B polymerase.

Accordingly, the present invention relates to the use of a compound of fomula (I) or any subgroup thereof as defined above for the manufacture of a medicament useful for inhibiting HCV activity in a mammal infected with HCV viruses, wherein said medicament is used in a combination therapy, said combination therapy preferably comprising a compound of formula (I) and (pegylated) IFN-α and/or ribavirin, and possibly an anti-HIV compound.

In addition to the presently found HCV antiviral activity, their known inhibitory activity against the HIV protease enzyme renders the compounds of the present invention useful in the treatment of individuals co-infected with HCV and HIV and for the prophylaxis of these individuals.

Therefore, HCV infected patients also suffering from conditions associated with HIV or even other pathogenic retroviruses, such as AIDS, AIDS-related complex (ARC), progressive generalized lymphadenopathy (PGL), as well as chronic CNS diseases caused by retroviruses, such as, for example HIV mediated dementia and multiple sclerosis, can conveniently be treated with the present compounds.

The compounds of the present invention or any subgroup thereof may therefore be used as medicines against above-mentioned conditions. Said use as a medicine or method of treatment comprises the systemic administration to patients co-infected with HIV and HCV of an amount effective to combat the conditions associated with HCV and HIV. Consequently, the compounds of the present invention can be used in the manufacture of a medicament useful for treating conditions associated with the co-infection of HCV and HIV, including multi-drug resistant HIV virus.

In another embodiment, the present invention relates to the use of formula (I) or any subgroup thereof in the manufacture of a medicament useful for inhibiting HCV activity and HIV activity, and in particular inhibiting a protease of a multi-drug resistant HIV-1 retrovirus, in a mammal infected with both HCV and HIV.

For an oral administration form, compounds of the present invention are mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms.

For subcutaneous or intravenous administration, the active compounds, if desired with the substances customary therefor such as solubilizers, emulsifiers or further auxiliaries, are brought into solution, suspension, or emulsion. The compounds of formula (I) can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned.

Suitable pharmaceutical formulations for administration in the form of aerosols or sprays are, for example, solutions, suspensions or emulsions of the compounds of formula (I) or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant. Such a preparation customarily contains the active compound in a concentration from approximately 0.1 to 50%, in particular from approximately 0.3 to 3% by weight.

Another aspect of the present invention concerns a kit or container comprising a compound of formula (I) in an amount effective for use as a standard or reagent in a test or assay for determining the ability of a potential pharmaceutical to inhibit HCV growth, and possibly also to inhibit HIV growth and/or HIV protease activity. This aspect of the invention may find its use in pharmaceutical research programs.

The compounds of the present invention can be used in high-throughput target-analyte assays such as those for measuring the efficacy of said compound in HCV or combined HCV/HIV treatment.

The dose of the present compounds or of the physiologically tolerable salt(s) thereof to be administered depends on the individual case and, as customary, is to be adapted to the conditions of the individual case for an optimum effect. Thus it depends, of course, on the frequency of administration and on the potency and duration of action of the compounds employed in each case for therapy or prophylaxis, but also on the nature and severity of the infection and symptoms, and on the sex, age, weight and individual responsiveness of the human or animal to be treated and on whether the therapy is acute or prophylactic. Customarily, the daily dose of a compound of formula (I) in the case of administration to a patient approximately 75 kg in weight is 1 mg to 1.5g, preferably 10 mg to 1 g. The dose can be administered in the form of an individual dose, or divided into several, e.g. two, three, or four, individual doses.

Accordingly, another aspect of the present invention concerns a kit or container comprising a compound of formula (I) in an amount effective to combat, treat or ameliorate the conditions associated with HCV infection and/or HCV/HIV coinfection in a mammal.

It will be appreciated by the person skilled in the art that the compounds of formula (I) may be tested in a cellular HCV replicon system based on Lohmann et al. (1999) Science 285:110-113, with the further modifications described by Krieger et al. (2001) Journal of Virology 75: 4614-4624 (incorporated herein by reference), which is further exemplified in the examples section. This model, while not a complete infection model for HCV, is widely accepted as the most robust and efficient model of autonomous HCV RNA replication currently available. Compounds exhibiting anti-HCV activity in this cellular model are considered as candidates for further development in the treatment of HCV infections in mammals. It will be appreciated that it is important to distinguish between compounds that specifically interfere with HCV functions from those that exert cytotoxic or cytostatic effects in the HCV replicon model, and as a consequence cause a decrease in HCV RNA or linked reporter enzyme concentration. Assays are known in the field for the evaluation of cellular cytotoxicity based for example on the activity of mitochondrial enzymes using fluorogenic redox dyes such as resazurin. Furthermore, cellular counterscreens exist for the evaluation of non-selective inhibition of linked reporter gene activity, such as firefly luciferase. Appropriate cell types can be equipped by stable transfection with a luciferase reporter gene whose expression is dependent on a constitutively active gene promoter, and such cells can be used as a counterscreen to eliminate non-selective inhibitors.

All patents, patent applications and articles referred to before or below are incorporated herein by reference.

### Examples section

### Example 1: Activity of compounds of formula (I)in HCV replicon assays

### Stable replicon cell reporter assays:

The compounds of the present invention were examined for activity in the inhibition of HCV RNA replication in a cellular assay. The assay demonstrated that these compounds exhibited activity against HCV replicons functional in a cell culture. The cellular assay was based on a bicistronic expression construct, as described by Lohmann et al. (1999) Science vol. 285 pp. 110-113 with modifications described by Krieger et al. (2001) Journal of Virology 75: 4614-4624, in a multi-target screening strategy. In essence, the method is as follows.

The assay utilized the stably transfected cell line Huh-7 luc/neo (hereafter referred to as Huh-Luc; kindly provided by Dr Ralf Bartenschlager, Reblikon GmBh, Germany). This cell line harbors an RNA encoding a bicistronic expression construct comprising the wild type NS3-NS5B regions of HCV type 1b translated from an Internal Ribosome Entry Site (IRES) from encephalomyocarditis virus (EMCV), preceded by a reporter portion (FfL-luciferase), and a selectable marker portion (neo^{R}, neomycine phosphotransferase). The construct was bordered by 5' and 3' NTRs (non-translated regions) from HCV type 1b. Continued culture of the replicon cells in the presence of G418 (neo^{R}) is dependent on the replication of the HCV RNA.The stably transfected replicon cells that express HCV RNA, which replicates autonomously and to high levels, encoding *inter alia* luciferase, were used for screening the antiviral compounds.

### Cellular Assay Experimental Method:

The replicon cells were plated in 384 well plates in the presence of the test and control compounds which were added in various concentrations. Following an incubation of three days, HCV replication was measured by assaying luciferase activity (using standard luciferase assay substrates and reagents and a Perkin Elmer ViewLux^{Tm} ultraHTS microplate imager). Replicon cells in the control cultures have high luciferase expression in the absence of any inhibitor. The inhibitory activity of the compound on luciferase activity was monitored on the Huh-Luc cells, enabling a dose-response curve for each test compound. EC50 values were then calculated, which value represents the amount of the compound required to decrease by 50% the level of detected luciferase activity, or more specifically, the ability of the genetically linked HCV replicon RNA to replicate (Table 1).

In table 1, column 1 provides an identification number, column 2 displays the results as EC50 on the above described Huh7-Luc assay wherein:
++++ means an EC50 less than 2 µM
+++ means an EC50 between 2 and 10 µM
++ means an EC50 between 10 and 32 µM
+ means an EC50 greater than 32 µM.

Column 5 provides the chemical structure of the compounds tested.

| Compound | EC50 value | Compound Name |
|---|---|---|
| 1 | +++ | {3-[(2-Acetylamino-benzooxazole-6-sulfonyl)-isobutyl-amino]-1-benzyl-2-hydroxy-propyl}-carbamic acid thiazol-5-ylmethyl ester |
| 2 | ++ | (6-{[2-Hydroxy-4-phenyl-3-(thiazol-5-ylmethoxycarbonyl-amino)-butyl]-isobutyl-sulfamoyl}-benzooxazol-2-yl)-carbamic acid ethyl ester |
| 3 | ++++ | [1-Benzyl-2-hydroxy-3-({2-[(6-hydroxy-pyridine-3-carbonyl)-amino]-benzooxazole-6-sulfonyl}-isobutyl-amino)-propyl]- |
| 4 | +++ | carbamic acid thiazol-5-ylmethyl ester [1-Benzyl-2-hydroxy-3-(isobutyl-{2-[(pyridine-3-carbonyl)-amino]-benzooxazole-6-sulfonyl}-amino)-propyl]-carbamic acid thiazol-5-ylmethyl ester |
| 5 | +++ | {1-Benzyl-2-hydroxy-3-[isobutyl-(2-pyrrolidin-1-yl-benzo-oxazole-6-sulfonyl)-amino]-propyl}-carbamic acid thiazol-5-ylmethyl ester |
| 6 | ++ | [1-Benzyl-2-hydroxy-3-(isobutyl-{2-[methyl-(2-pyrrolidin-1-yl-ethyl)-amino]-benzooxazole-6-sulfonyl}-amino)-propyl]-carbamic acid thiazol-5-ylmethyl ester |
| 7 | +++ | [1-Benzyl-2-hydroxy-3-(isobutyl-{2-[2-(4-methyl-piperazin-1-yl)-acetylamino]-benzooxazole-6-sulfonyl}-amino)-propyl]-carbamic acid thiazol-5-ylmethyl ester |
| 8 | ++ | [1-Benzyl-2-hydroxy-3-(isobutyl-{2-[methyl-(5-oxo-pyrrolidine-2-carbonyl)-amino]-benzooxazole-6-sulfonyl}-amino)-propyl]-carbamic acid thiazol-5-ylmethyl ester |
| 9 | ++ | [1-Benzyl-2-hydroxy-3-(isobutyl-{2-[methyl-(pyridine-4-carbonyl)-amino]-benzooxazole-6-sulfonyl}-amino)-propyl]-carbamic acid thiazol-5-ylmethyl ester |
| 10 | +++ | [1-Benzyl-3-({2-[(furan-3-carbonyl)-methyl-amino]-benzo-oxazole-6-sulfonyl}-isobutyl-amino)-2-hydroxy-propyl]-carbamic acid thiazol-5-ylmethyl ester |
| 11 | +++ | [1-Benzyl-2-hydroxy-3-(isobutyl-{2-[(1-methyl-pyrrolidine-2-carbonyl)-amino]-benzooxazole-6-sulfonyl}-amino)-propyl]-carbamic acid thiazol-5-ylmethyl ester |
| 12 | +++ | {1-Benzyl-3-[(3-benzyl-3H-benzoimidazole-5-sulfonyl)-isobutyl-amino]-2-hydroxy-propyl}-carbamic acid thiazol-5-ylmethyl ester |
| 13 | +++ | {3-[(2-Amino-benzothiazole-6-sulfonyl)-isobutyl-amino]-1-benzyl-2-hydroxy-propyl}-carbamic acid thiazol-5-ylmethyl ester |
| 14 | +++ | (1-Benzyl-3-{[2-(2-dimethylamino-ethylamino)-benzo-thiazole-6-sulfonyl]-isobutyl-amino}-2-hydroxy-propyl)-carbamic acid thiazol-5-ylmethyl ester |
| 15 | +++ | (1-Benzyl-2-hydroxy-3-{isobutyl-[2-(2-pyrrolidin-1-yl-ethyl-amino)-benzothiazole-6-sulfonyl]-amino}-propyl)-carbamic acid thiazol-5-ylmethyl ester |
| 16 | +++ | (1-Benzyl-2-hydroxy-3-{isobutyl-[2-(2-pyrrolidin-1-yl-ethylamino)-benzothiazole-6-sulfonyl]-amino}-propyl)-carbamic acid thiazol-5-ylmethyl ester trifluoroacetate salt |
| 17 | ++ | (1-Benzyl-3-{[2-(3-dimethylamino-propylamino)-benzo-thiazole-6-sulfonyl]-isobutyl-amino}-2-hydroxy-propyl)-carbamic acid thiazol-5-ylmethyl ester |
| 18 | +++ | (1-Benzyl-2-hydroxy-3-{isobutyl-[2-(2-piperazin-1-yl-ethyl-amino)-benzothiazole-6-sulfonyl]-amino}-propyl)-carbamic acid thiazol-5-ylmethyl ester |
| 19 | +++ | {3-[(2-Amino-benzooxazole-6-sulfonyl)-isobutyl-amino]-1-benzyl-2-hydroxy-propyl}-carbamic acid thiazol-5-ylmethyl ester |
| 20 | ++ | {3-[(3H-Benzoimidazole-5-sulfonyl)-isobutyl-amino]-1-benzyl-2-hydroxy-propyl}-carbamic acid thiazol-5-ylmethyl ester |
| 21 | ++ | (3-{[2-(Acetyl-methyl-amino)-benzothiazole-6-sulfonyl]-isobutyl-amino}-1-benzyl-2-hydroxy-propyl)-carbamic acid thiazol-5-ylmethyl ester |
| 22 | + | {3-[(2-Amino-benzooxazole-6-sulfonyl)-pyridin-2-ylmethyl-amino]-1-benzyl-2-hydroxy-propyl}-carbamic acid thiazol-5-ylmethyl ester trifluoroacetate salt |
| 23 | ++ | [1-Benzyl-2-hydroxy-3-(isobutyl-{2-[(5-oxo-pyrrolidine-2-carbonyl)-amino]-benzooxazole-6-sulfonyl}-amino)-propyl]-carbamic acid thiazol-5-yl methyl ester |
| 24 | +++ | [1-Benzyl-2-hydroxy-3-(isobutyl-{2-[(5-oxo-pyrrolidine-2-carbonyl)-amino]-benzooxazole-6-sulfonyl}-amino)-propyl]-carbamic acid thiazol-5-yl methyl ester |
| 25 | ++++ | [1-Benzyl-3-({2-[(furan-3-carbonyl)-amino]-benzooxazole-6-sulfonyl}-isobutyl-amino)-2-hydroxy-propyl]-carbamic acid thiazol-5-ylmethyl ester |
| 26 | ++ | [1-Benzyl-2-hydroxy-3-(isobutyl-{2-[(1-methyl-piperidine-4-carbonyl)-amino]-benzooxazole-6-sulfonyl}-amino)-propyl]-carbamic acid thiazol-5-ylmethyl ester |
| 27 | +++ | [1-Benzyl-2-hydroxy-3-(isobutyl-{2-[(pyridine-2-carbonyl)-amino]-benzooxazole-6-sulfonyl}-amino)-propyl]-carbamic acid thiazol-5-ylmethyl ester |
| 28 | +++ | {3-[(2-Amino-benzooxazole-6-sulfonyl)-isobutyl-amino]-1-benzyl-2-hydroxy-propyl}-carbamic acid 2-chloro-thiazol-5-ylmethyl ester |
| 29 | +++ | (1-Benzyl-3-{[2-(2-dimethylamino-acetylamino)-benzo-oxazole-6-sulfonyl]-isobutyl-amino}-2-hydroxy-propyl)-carbamic acid thiazol-5-ylmethyl ester |
| 30 | +++ | {1-Benzyl-2-hydroxy-3-[isobutyl-(2-piperazin-1-yl-benzo-oxazole-6-sulfonyl)-amino]-propyl}-carbamic acid thiazol-5-ylmethyl ester |
| 31 | +++ | {1-Benzyl-2-hydroxy-3-[isobutyl-(2-piperidin-1-yl-benzo-oxazole-6-sulfonyl)-amino]-propyl}-carbamic acid thiazol-5-ylmethyl ester |
| 32 | ++ | {1-Benzyl-2-hydroxy-3-[isobutyl-(2-{2-[methyl-(2-pyrrolidin-1-yl-ethyl)-amino]-acetylamino}-benzooxazole-6-sulfonyl)-amino]-propyl}-carbamic acid thiazol-5-ylmethyl ester |
| 33 | ++ | {1-Benzyl-3-[(2-dimethylamino-benzooxazole-6-sulfonyl)-isobutyl-amino]-2-hydroxy-propyl}-carbamic acid thiazol-5-ylmethyl ester |
| 34 | ++ | {3-[(2-Amino-benzooxazole-6-sulfonyl)-isobutyl-amino]-1-benzyl-2-hydroxy-propyl}-carbamic acid oxazol-5-ylmethyl ester |
| 35 | ++++ | [1-Benzyl-2-hydroxy-3-(isobutyl-{2-[(pyridine-4-carbonyl)-amino]-benzooxazole-6-sulfonyl}-amino)-propyl]-carbamic acid thiazol-5-ylmethyl ester |
| 36 | ++ | [1-Benzyl-2-hydroxy-3-(isobutyl-{2-[methyl-(pyridine-2-carbonyl)-amino]-benzooxazole-6-sulfonyl}-amino)-propyl]-carbamic acid thiazol-5-ylmethyl ester |
| 37 | ++ | [1-Benzyl-2-hydroxy-3-(isobutyl-{2-[methyl-(1-methyl-piperidine-3-carbonyl)-amino]-benzooxazole-6-sulfonyl}-amino)-propyl]-carbamic acid thiazol-5-ylmethyl ester |
| 38 | ++ | [1-Benzyl-2-hydroxy-3-(isobutyl-{2-[methyl-(1-methyl-piperidine-4-carbonyl)-amino]-benzooxazole-6-sulfonyl}-amino)-propyl]-carbamic acid thiazol-5-ylmethyl ester |
| 39 | ++ | [1-Benzyl-3-({2-[(2-chloro-pyridine-4-carbonyl)-methyl-amino]-benzooxazole-6-sulfonyl}-isobutyl-amino)-2-hydroxy-propyl]-carbamic acid thiazol-5-ylmethyl ester |
| 40 | ++ | [1-Benzyl-2-hydroxy-3-(isobutyl-{2-[methyl-(1-methyl-pyrrolidine-2-carbonyl)-amino]-benzooxazole-6-sulfonyl}-amino)-propyl]-carbamic acid thiazol-5-ylmethyl ester trifluoroacetate salt |
| 41 | ++ | {1-Benzyl-2-hydroxy-3-[isobutyl-(3-phenethyl-3H-benzo-imidazole-5-sulfonyl)-amino]-propyl}-carbamic acid thiazol-5-ylmethyl ester |
| 42 | +++ | {1-Benzyl-2-hydroxy-3-[isobutyl-(3-isobutyl-3H-benzo-imidazole-5-sulfonyl)-amino]-propyl}-carbamic acid thiazol-5-ylmethyl ester |
| 43 | ++++ | [1-Benzyl-2-hydroxy-3-(isobutyl-{4-[2-(pyridin-4-ylamino)-thiazol-4-yl]-benzenesulfonyl}-amino)-propyl]-carbamic acid thiazol-5-ylmethyl ester |
| 44 | ++ | (1-Benzyl-2-hydroxy-3-{isobutyl-[4-(2-methyl-oxazol-4-yl)-benzenesulfonyl]-amino}-propyl)-carbamic acid thiazol-5-ylmethyl ester |
| 45 | +++ | {3-[(2-Amino-benzooxazole-6-sulfonyl)-isobutyl-amino]-1-benzyl-2-hydroxy-propyl}-carbamic acid thiazol-5-ylmethyl ester |

### Example 2: Dose preparations of the compounds of formula (I)

### Formulation

Active ingredient, *in casu* a compound of formula (I), is dissolved in organic solvent such as ethanol, methanol or methylene chloride, preferably, a mixture of ethanol and methylene chloride. Polymers such as polyvinylpyrrolidone copolymer with vinyl acetate (PVP-VA) or hydroxypropylmethylcellulose (HPMC), typically 5 mPa.s, are dissolved in organic solvents such as ethanol, methanol methylene chloride. Suitably the polymer is dissolved in ethanol. The polymer and compound solutions are mixed and subsequently spray dried. The ratio of compound/polymer is selected from 1/1 to 1/6. Intermediate ranges are 1/1.5 and 1/3. A suitable ratio is 1/6. The spray-dried powder, a solid dispersion, is subsequently filled in capsules for administration. The drug load in one capsule ranges between 50 and 100 mg depending on the capsule size used.

### Film-coated Tablets

### Preparation of Tablet Core

A mixture of 100 g of active ingredient, *in casu* a compound of formula (I), 570 g lactose and 200 g starch is mixed well and thereafter humidified with a solution of 5 g sodium dodecyl sulfate and 10 g polyvinylpyrrolidone in about 200 ml of water. The wet powder mixture is sieved, dried and sieved again. Then there is added 100 g microcrystalline cellulose and 15 g hydrogenated vegetable oil. The whole is mixed well and compressed into tablets, giving 10.000 tablets, each comprising 10 mg of the active ingredient.

### Coating

To a solution of 10 g methylcellulose in 75 ml of denaturated ethanol there is added a solution of 5 g of ethylcellulose in 150 ml of dichloromethane. Then there are added 75 ml of dichloromethane and 2.5 ml 1,2,3-propanetriol. 10 g of polyethylene glycol is molten and dissolved in 75 ml of dichloromethane. The latter solution is added to the former and then there are added 2.5 g of magnesium octadecanoate, 5 g of polyvinylpyrrolidone and 30 ml of concentrated color suspension and the whole is homogenated The tablet cores are coated with the thus obtained mixture in a coating apparatus.

## Claims

1. The use of sulfonamide derivatives having the general formula 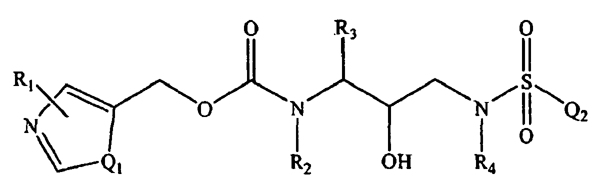 or a *N*-oxide, salt, stereoisomeric form, racemic mixture, prodrug or esters thereof, wherein
Q₁ is -S- or -O-;
R₁ is hydrogen, C₁₋₆alkyl, hydroxy, amino, halogen, aminoC₁₋₄alkyl and mono-or di(C₁₋₄alkyl)amino;
R₂, R₁₄ and R₁₅ are, each independently, hydrogen or C₁₋₆alkyl;
R₃ is C₁₋₆alkyl, aryl, C₃₋₇cycloalkyl, C₃₋₇cycloalkylC₁₋₄alkyl, or arylC₁₋₄alkyl;
R₄ is hydrogen, C₁₋₄alkyloxycarbonyl, carboxyl, optionally mono- or disubstituted aminocarbonyl, mono- or di(C₁₋₄alkyl)aminocarbonyl, C₃₋₇cycloalkyl, C₂₋₆alkenyl, C₂₋₆alkynyl or C₁₋₆alkyl optionally substituted with one or more substituents each independently selected from aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkylS(=O)ₜ, hydroxy, cyano, halogen or amino optionally mono- or di-substituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl;
Q₂ is a radical of formula (III), (IV), (V), (VI), or (VII) 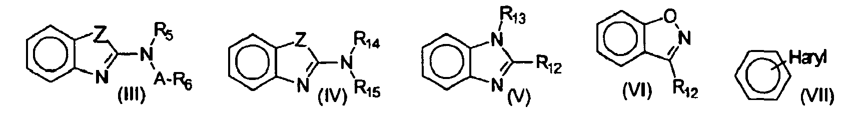 and is be attached to the remainder of the molecule via any available carbon atom of the phenyl or fused phenyl ring,
Z is O or S;
A is C₁₋₆alkanediyl, -C(=O)-, -C(=S)-, -S(=O)₂-, C₁₋₆alkanediyl-C(=O)-, C₁₋₆alkanediyl-C(=S)- or C₁₋₆alkanediyl-S(=O)₂-; wherein the point of attachment to the nitrogen atom is the C₁₋₆alkanediyl group in those moieties containing said group;
R₅ is hydrogen, hydroxy, C₁₋₆alkyl, Het¹C₁₋₆alkyl, Het²C₁₋₆alkyl, or aminoC₁₋₆alkyl wherein the amino goup may optionally be mono- or di-substituted with C₁₋₄alkyl;
R₆ is C₁₋₆alkyloxy, Het¹, Het¹oxy, Het², Het²oxy, aryl, aryloxy or amino; and in case -A- is other than C₁₋₆alkanediyl then R₆ may also be C₁₋₆alkyl, Het¹C₁₋₄-alkyl, Het¹oxyC₁₋₄alkyl, Het²C₁₋₄alkyl, Het²oxyC₁₋₄alkyl, arylC₁₋₄alkyl, aryloxyC₁₋₄alkyl or aminoC₁₋₄alkyl; wherein each of the amino groups in the definition of R₆ may optionally be substituted with one or more substituents selected from C₁₋₄alkyl, C₁₋₄alkylcarbonyl, C₁₋₄alkyloxycarbonyl, aryl, arylcarbonyl, aryloxycarbonyl, Het¹, Het², arylC₁₋₄alkyl, Het¹C₁₋₄alkyl or Het²C₁₋₄alkyl;; and
R₅ and -A-R₆ taken together with the nitrogen atom to which they are attached may also form Het¹ or Het²;
R₁₂ is hydrogen, -NH₂, -N(R₅)(AR₆), -C₁₋₆alkyl or C₁₋₆alkyl-W-R₁₇, wherein each C₁₋₆alkyl may optionally be substituted with halogen, hydroxy, aryl, Het¹, Het², amino or mono- or di-(C₁₋₄ alkyl)amino;
W is oxy, carbonyl, oxycarbonyl, carbonyloxy, oxycarbonyloxy, amino, aminocarbonyl, carbonylamino or sulphur;
R₁₃ is hydrogen or C₁₋₆-alkyl optionally substituted with a substituent selected from the group consisting of aryl, Het¹, Het², hydroxy, halogen or amino, wherein the amino group may be optionally be mono- or di-substituted with C₁₋₄alkyl;
R₁₇ is C₁₋₆alkyl, aryl, Het¹ or Het²;
Haryl is an aromatic monocyclic, bicyclic or tricyclic heterocycle having 3 to 14 ring members which contains one or more heteroatom ring members selected from nitrogen, oxygen and sulfur and which may optionally be substituted on (i) one or more carbon atoms by a substituent selected from the group consisting of C₁₋₆alkyl, halogen, hydroxy, optionally mono- or di-substituted amino, nitro, cyano, haloC₁₋₆alkyl, carboxyl, C₃₋₇cycloalkyl, optionally mono- or disubstituted aminocarbonyl, methylthio, methylsulfonyl, aryl, -(R₇ₐ)ₙ-M-R_{7b}, Het¹ and Het²; wherein the optional substituents on any amino function in the above group of substituents are independently selected from R₅ and -A-R₆; and on (ii) a nitrogen atom if present by hydroxy or -A-R₆;
R₇ₐ is C₁₋₆alkanediyl optionally substituted with one or more substituents selected from, halogen, C₁₋₄alkylcarbonyl, C₁₋₄alkyloxycarbonyl, aryl, arylcarbonyl, aryloxycarbonyl, Het¹ or Het²;
R_{7b} is C₁₋₆alkyl optionally substituted with one or more substituents selected from halogen, C₁₋₄alkylcarbonyl, C₁₋₄alkyloxycarbonyl, aryl, arylcarbonyl, aryloxycarbonyl, Het¹ or Het²;
R₈ is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het² or Het²C₁₋₆alkyl;
M is defined by -C(=O)-, -O-C(=O)-, -C(=O)-O-, -CH₂-CHOH-, -CHOH-CH₂-, -NR₈-C(=O)-, -(C=O)-NR₈-, -S(=O)₂-, -O-, -S-, -O-S(=O)₂-, -S(=O)₂-O-, -NR₈-S(=O)₂ or -S(=O)₂-NR₈-;
n is zero or 1;
for the manufacture of a medicament useful for inhibiting HCV activity in a mammal infected with HCV.

2. The use as claimed in claim 1 wherein Q₂ is a radical of formula (III).

3. The use as claimed in claim 1 wherein Q₂ is a radical of formula (IV).

4. The use as claimed in claim 1 wherein Q₂ is a radical of formula (V).

5. The use as claimed in claim 1 wherein Q₂ is a radical of formula (VI).

6. The use as claimed in claim 1 wherein Q₂ is a radical of formula (VII).

7. The use as claimed in claim 2 wherein A is -C(=O)- or C₁₋₆alkanediyl, R₅ is hydrogen or C₁₋₆alkyl; or taken together with -A-R₆ and with the nitrogen atom to which it is attached forms a Het¹; R₆ is C₁₋₆alkyloxy, Het¹, Het², aryl or amino; and in case -A- is other than C₁₋₆alkanediyl then R₆ may also be C₁₋₆alkyl, Het¹C₁₋₄-alkyl, Het²C₁₋₄alkyl, arylC₁₋₄alkyl or aminoC₁₋₄alkyl; wherein each of the amino groups in the definition of R₆ may optionally be substituted with one or more substituents selected from C₁₋₄alkyl, arylC₁₋₄alkyl, Het¹C₁₋₄alkyl or Het²C₁₋₄alkyl.

8. The use as claimed in claim 3 wherein R¹⁴ and R¹⁵ are both hydrogen or are both methyl.

9. The use as claimed in claim 4 wherein R₁₂ is hydrogen and R₁₃ is hydrogen or C₁₋₆-alkyl optionally substituted with aryl.

10. The use as claimed in claim 6 wherein Haryl is thiazolyl or oxazolyl which may both optionally be substituted with C₁₋₆alkyl or Het²amino.

11. The use as claimed in any one of claims 1 to 10 wherein R₂ is hydrogen, R₃ is arylC₁₋₄alkyl and R₄ is C₁₋₆alkyl.

12. The use as claimed in claim 1 wherein the compound is
{3-[(2-Acetylamino-benzooxazole-6-sulfonyl)-isobutyl-amino]-1-benzyl-2-hydroxy-propyl}-carbamic acid thiazol-5-ylmethyl ester;
(6-{[2-Hydroxy-4-phenyl-3-(thiazol-5-ylmethoxycarbonylamino)-butyl]-isobutyl-sulfamoyl}-benzooxazol-2-yl)-carbamic acid ethyl ester;
[1-Benzyl-2-hydroxy-3-({2-[(6-hydroxy-pyridine-3-carbonyl)-amino]-benzooxazole-6-sulfonyl}-isobutyl-amino)-propyl]-carbamic acid thiazol-5-ylmethyl ester;
[1-Benzyl-2-hydroxy-3-(isobutyl-{2-[(pyridine-3-carbonyl)-amino]-benzooxazole-6-sulfonyl}-amino)-propyl]-carbamic acid thiazol-5-ylmethyl ester;
{1-Benzyl-2-hydroxy-3-[isobutyl-(2-pyrrolidin-1-yl-benzooxazole-6-sulfonyl)-amino]-propyl}-carbamic acid thiazol-5-ylmethyl ester;
[1-Benzyl-2-hydroxy-3-(isobutyl-{2-[methyl-(2-pyrrolidin-1-yl-ethyl)-amino]-benzooxazole-6-sulfonyl}-amino)-propyl]-carbamic acid thiazol-5-ylmethyl ester;
[1-Benzyl-2-hydroxy-3-(isobutyl-{2-[2-(4-methyl-piperazin-1-yl)-acetylamino]-benzooxazole-6-sulfonyl}-amino)-propyl]-carbamic acid thiazol-5-ylmethyl ester;
[1-Benzyl-2-hydroxy-3-(isobutyl-{2-[methyl-(5-oxo-pyrrolidine-2-carbonyl)-amino]-benzooxazole-6-sulfonyl}-amino)-propyl]-carbamic acid thiazol-5-ylmethyl ester;
[1-Benzyl-2-hydroxy-3-(isobutyl-{2-[methyl-(pyridine-4-carbonyl)-amino]-benzooxazole-6-sulfonyl}-amino)-propyl]-carbamic acid thiazol-5-ylmethyl ester;
[1-Benzyl-3-({2-[(furan-3-carbonyl)-methyl-amino]-benzooxazole-6-sulfonyl}-isobutyl-amino)-2-hydroxy-propyl]-carbamic acid thiazol-5-ylmethyl ester;
[1-Benzyl-2-hydroxy-3-(isobutyl-{2-[(1-methyl-pyrrolidine-2-carbonyl)-amino]-benzooxazole-6-sulfonyl}-amino)-propyl]-carbamic acid thiazol-5-ylmethyl ester;
{1-Benzyl-3-[(3-benzyl-3H-benzoimidazole-5-sulfonyl)-isobutyl-amino]-2-hydroxy-propyl}-carbamic acid thiazol-5-ylmethyl ester;
{3-[(2-Amino-benzothiazole-6-sulfonyl)-isobutyl-amino]-1-benzyl-2-hydroxypropyl}-carbamic acid thiazol-5-ylmethyl ester;
(1-Benzyl-3-{[2-(2-dimethylamino-ethylamino)-benzothiazole-6-sulfonyl]-isobutyl-amino}-2-hydroxy-propyl)-carbamic acid thiazol-5-ylmethyl ester;
(1-Benzyl-2-hydroxy-3-{isobutyl-[2-(2-pyrrolidin-1-yl-ethylamino)-benzothiazole-6-sulfonyl]-amino}-propyl)-carbamic acid thiazol-5-ylmethyl ester;
(1-Benzyl-2-hydroxy-3-{isobutyl-[2-(2-pyrrolidin-1-yl-ethylamino)-benzothiazole-6-sulfonyl]-amino}-propyl)-carbamic acid thiazol-5-ylmethyl ester trifluoroacetate salt;
(1-Benzyl-3-{[2-(3-dimethylamino-propylamino)-benzothiazole-6-sulfonyl]-isobutyl-amino}-2-hydroxy-propyl)-carbamic acid thiazol-5-ylmethyl ester;
(1-Benzyl-2-hydroxy-3- {isobutyl-[2-(2-piperazin-1-yl-ethylamino)-benzothiazole-6-sulfonyl]-amino}-propyl)-carbamic acid thiazol-5-ylmethyl ester,
{3-[(2-Amino-benzooxazole-6-sulfonyl)-isobutyl-amino]-1-benzyl-2-hydroxypropyl}-carbamic acid thiazol-5-ylmethyl ester;
{3-[(3H-Benzoimidazole-5-sulfonyl)-isobutyl-amino]-1-benzyl-2-hydroxypropyl}-carbamic acid thiazol-5-ylmethyl ester;
(3- {[2-(Acetyl-methyl-amino)-benzothiazole-6-sulfonyl]-isobutyl-amino}-1-benzyl-2-hydroxy-propyl)-carbamic acid thiazol-5-ylmethyl ester;
{3-[(2-Amino-benzooxazole-6-sulfonyl)-pyridin-2-ylmethyl-amino]-1-benzyl-2-hydroxy-propyl}-carbamic acid thiazol-5-ylmethyl ester trifluoroacetate salt;
[1-Benzyl-2-hydroxy-3-(isobutyl-{2-[(5-oxo-pyrrolidine-2-carbonyl)-amino]-benzooxazole-6-sulfonyl}-amino)-propyl]-carbamic acid thiazol-5-yl methyl ester;
[1-Benzyl-2-hydroxy-3-(isobutyl-{2-[(5-oxo-pyrrolidine-2-carbonyl)-amino]-benzooxazole-6-sulfonyl}-amino)-propyl]-carbamic acid thiazol-5-yl methyl ester;
[1-Benzyl-3-({2-[(furan-3-carbonyl)-amino]-benzooxazole-6-sulfonyl}-isobutyl-amino)-2-hydroxy-propyl]-carbamic acid thiazol-5-ylmethyl ester;
[1-Benzyl-2-hydroxy-3-(isobutyl-{2-[(1-methyl-piperidine-4-carbonyl)-amino]-benzooxazole-6-sulfonyl}-amino)-propyl]-carbamic acid thiazol-5-ylmethyl ester;
[1-Benzyl-2-hydroxy-3-(isobutyl-{2-[(pyridine-2-carbonyl)-amino]-benzooxazole-6-sulfonyl}-amino)-propyl]-carbamic acid thiazol-5-ylmethyl ester;
{3-[(2-Amino-benzooxazole-6-sulfonyl)-isobutyl-amino]-1-benzyl-2-hydroxypropyl}-carbamic acid 2-chloro-thiazol-5-ylmethyl ester;
(1-Benzyl-3-{[2-(2-dimethylamino-acetylamino)-benzooxazole-6-sulfonyl]-isobutyl-amino}-2-hydroxy-propyl)-carbamic acid thiazol-5-ylmethyl ester;
{1-Benzyl-2-hydroxy-3-[isobutyl-(2-piperazin-1-yl-benzooxazole-6-sulfonyl)-amino]-propyl}-carbamic acid thiazol-5-ylmethyl ester;
{1-Benzyl-2-hydroxy-3-[isobutyl-(2-piperidin-1-yl-benzooxazole-6-sulfonyl)-amino]-propyl}-carbamic acid thiazol-5-ylmethyl ester;
{1-Benzyl-2-hydroxy-3-[isobutyl-(2-{2-[methyl-(2-pyrrolidin-1-yl-ethyl)-amino]-acetylamino}-benzooxazole-6-sulfonyl)-amino]-propyl}-carbamic acid thiazol-5-ylmethyl ester;
{1-Benzyl-3-[(2-dimethylamino-benzooxazole-6-sulfonyl)-isobutyl-amino]-2-hydroxy-propyl}-carbamic acid thiazol-5-ylmethyl ester;
{3-[(2-Amino-benzooxazole-6-sulfonyl)-isobutyl-amino]-1-benzyl-2-hydroxypropyl}-carbamic acid oxazol-5-ylmethyl ester;
[1-Benzyl-2-hydroxy-3-(isobutyl-{2-[(pyridine-4-carbonyl)-amino]-benzooxazole-6-sulfonyl}-amino)-propyl]-carbamic acid thiazol-5-ylmethyl ester;
[1-Benzyl-2-hydroxy-3-(isobutyl-{2-[methyl-(pyridine-2-carbonyl)-amino]-benzooxazole-6-sulfonyl}-amino)-propyl]-carbamic acid thiazol-5-ylmethyl ester;
[1-Benzyl-2-hydroxy-3-(isobutyl-{2-[methyl-(1-methyl-piperidine-3-carbonyl)-amino]-benzooxazole-6-sulfonyl}-amino)-propyl]-carbamic acid thiazol-5-ylmethyl ester;
[1-Benzyl-2-hydroxy-3-(isobutyl-{2-[methyl-(1-methyl-piperidine-4-carbonyl)-amino]-benzooxazole-6-sulfonyl}-amino)-propyl]-carbamic acid thiazol-5-ylmethyl ester;
[1-Benzyl-3-({2-[(2-chloro-pyridine-4-carbonyl)-methyl-amino]-benzooxazole-6-sulfonyl}-isobutyl-amino)-2-hydroxy-propyl]-carbamic acid thiazol-5-ylmethyl ester;
[1-Benzyl-2-hydroxy-3-(isobutyl-{2-[methyl-(1-methyl-pyrrolidine-2-carbonyl)-amino]-benzooxazole-6-sulfonyl}-amino)-propyl]-carbamic acid thiazol-5-ylmethyl ester trifluoroacetate salt;
{1-Benzyl-2-hydroxy-3-[isobutyl-(3-phenethyl-3H-benzoimidazole-5-sulfonyl)-amino]-propyl}-carbamic acid thiazol-5-ylmethyl ester;
{1-Benzyl-2-hydroxy-3-[isobutyl-(3-isobutyl-3H-benzoimidazole-5-sulfonyl)-amino]-propyl}-carbamic acid thiazol-5-ylmethyl ester;
[1-Benzyl-2-hydroxy-3-(isobutyl-{4-[2-(pyridin-4-ylamino)-thiazol-4-yl]-benzenesulfonyl}-amino)-propyl]-carbamic acid thiazol-5-ylmethyl ester;
(1-Benzyl-2-hydroxy-3-{isobutyl-[4-(2-methyl-oxazol-4-yl)-benzenesulfonyl]-amino}-propyl)-carbamie acid thiazol-5-ylmethyl ester or
{3-[(2-Amino-benzooxazole-6-sulfonyl)-isobutyl-amino]-1-benzyl-2-hydroxypropyl}-carbamic acid thiazol-5-ylmethyl ester,
or a N-oxide, salt, stereoisomeric form thereof.

13. The use as claimed in any one of claims 1 to 12 wherein the mammal is co-infected with HIV and HCV.

14. The use of a sulfonamide as defined in any one of claim 1 to 12 in the manufacture of a pharmaceutical composition aimed to treat or combat HCV infection.

15. A combination of a sulfonamide as defined in any one of claim 1 to 12 with another anti-HCV agent.

16. A combination as claimed in claim 15 further comprising an anti-HIV agent.

## Patentansprüche

1. Verwendung von Sulfonamidderivaten mit der allgemeinen Formel (I) 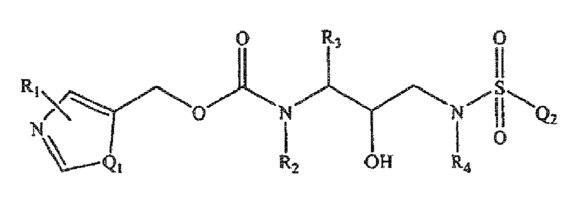 oder eines N-Oxids, Salzes, einer stereoisomeren Form, eines racemischen Gemischs, einer Arzneistoffvorstufe oder Estern davon, wobei:
Q₁ für -S- oder -O- steht;
R₁ für Wasserstoff, C₁₋₆-Alkyl, Hydroxy, Amino, Halogen, Amino-C₁₋₄-alkyl und Mono- oder Di(C₁₋₄-alkyl)amino steht;
R₂, R₁₄ und R₁₅ jeweils unabhängig für Wasserstoff oder C₁₋₆-Alkyl stehen;
R₃ für C₁₋₆-Alkyl, Aryl, C₃₋₇-Cycloalkyl, C₃₋₇-Cycloalkyl-C₁₋₄-alkyl oder Aryl-C₁₋₄-alkyl steht;
R₄ für Wasserstoff, C₁₋₄-Alkyloxycarbonyl, Carboxyl, gegebenenfalls einfach oder zweifach substituiertes Aminocarbonyl, Mono- oder Di(C₁₋₄-alkyl)aminocarbonyl, C₃₋₇-Cycloalkyl, C₂₋₆-Alkenyl, C₂₋₆-Alkinyl oder C₁₋₆-Alkyl gegebenenfalls substituiert mit einem oder mehreren Substituenten, jeweils unabhängig ausgewählt aus Aryl, Het¹, Het², C₃₋₇-Cycloalkyl, C₁₋₄-Alkyloxycarbonyl, Carboxyl, Aminocarbonyl, Mono- oder Di(C₁₋₄-alkyl)aminocarbonyl, Aminosulfonyl, C₁₋₄-Alkyl-S(=O)ₜ, Hydroxy, Cyano, Halogen oder gegebenenfalls einfach oder zweifach substituiertes Amino, worin die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-Alkyl und Het²-C₁₋₄-Alkyl steht;
Q₂ für einen Rest der Formel (III), (IV), (V), (VI) oder (VII) 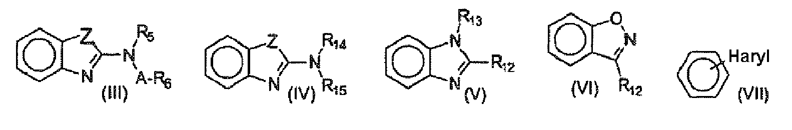 steht und an den Rest des Moleküls über ein beliebiges verfügbares Kohlenstoffatom des Phenyl- oder kondensierten Phenylrings gebunden wird,
Z für O oder S steht;
A für C₁₋₆-Alkandiyl, -C(=O)-, -C(=S)-, -S(=O)₂-, C₁₋₆-Alkandiyl-C(=O)-, C₁₋₆-Alkandiyl-C(=S)- oder C₁₋₆-Alkandiyl-S(=O)₂- steht, wobei in den Gruppierungen mit der C₁₋₆-Alkandiylgruppe diese den Punkt der Bindung an das Stickstoffatom darstellt;
R₅ für Wasserstoff, Hydroxy, C₁₋₆-Alkyl, Het¹-C₁₋₄-Alkyl, Het²-C₁₋₄-Alkyl oder Amino-C₁₋₆-alkyl, wobei die Aminogruppe gegebenenfalls einfach oder zweifach mit C₁₋₄-Alkyl substituiert sein kann, steht;
R₆ für C₁₋₆-Alkyloxy, Het¹, Het¹-Oxy, Het², Het²-Oxy, Aryl, Aryloxy oder Amino steht und, falls -A- nicht C₁₋₆-Alkandiyl ist, auch für C₁₋₆-Alkyl, Het¹-C₁₋₄-Alkyl, Het¹-Oxy-C₁₋₄-alkyl, Het²-C₁₋₄-Alkyl, Het²-Oxy-C₁₋₄-alkyl, Aryl-C₁₋₄-alkyl, Aryloxy-C₁₋₄-alkyl oder Amino-C₁₋₄-alkyl stehen kann, wobei die Aminogruppen in der Definition von R₆ jeweils gegebenenfalls mit einem oder mehreren Substituenten, ausgewählt aus C₁₋₄-Alkyl, C₁₋₄-Alkylcarbonyl, C₁₋₄-Alkyloxycarbonyl, Aryl, Arylcarbonyl, Aryloxycarbonyl, Het¹, Het², Aryl-C₁₋₄-alkyl, Het¹-C₁₋₄-Alkyl oder Het²-C₁₋₄-Alkyl, substituiert sein kann, und
R₅ und -A-R₆ zusammengenommen mit dem Stickstoffatom, an das sie gebunden sind, auch Het¹ oder Het² bilden können;
R₁₂ für Wasserstoff, -NH₂, -N(R₅)(AR₆), C₁₋₆-Alkyl oder C₁₋₆-Alkyl-W-R₁₇ stehen kann, wobei C₁₋₆-Alkyl jeweils gegebenenfalls mit Halogen, Hydroxy, Aryl, Het¹, Het², Amino oder Mono- oder Di(C₁₋₄-alkyl)amino substituiert sein kann;
W für Oxy, Carbonyl, Oxycarbonyl, Carbonyloxy, Oxycarbonyloxy, Amino, Aminocarbonyl, Carbonylamino oder Schwefel steht;
R₁₃ für Wasserstoff oder gegebenenfalls mit einem Substituenten, ausgewählt aus der Gruppe Aryl, Het¹, Het², Hydroxy, Halogen oder Amino, substituiertes C₁₋₆-Alkyl steht, wobei die Aminogruppe gegebenenfalls einfach oder zweifach mit C₁₋₄-Alkyl substituiert sein kann;
R₁₇ für C₁₋₆-Alkyl, Aryl, Het¹ oder Het² steht;
Haryl für einen aromatischen monocyclischen, bicyclischen oder tricyclischen Heterocyclus mit 3 bis 14 Ringgliedern steht, der einen oder mehrere Heteroatomringglieder, ausgewählt aus Stickstoff, Sauerstoff und Schwefel, enthält und der gegebenenfalls an (i) einem oder mehreren Kohlenstoffatomen mit einem Substituenten, ausgewählt aus der Gruppe C₁₋₆-Alkyl, Halogen, Hydroxy, gegebenenfalls einfach oder zweifach substituiertes Amino, Nitro, Cyano, Halogen-C₁₋₆-alkyl, Carboxyl, C₃₋₇-Cycloalkyl, gegebenenfalls einfach oder zweifach substituiertes Aminocarbonyl, Methylthio, Methylsulfonyl, Aryl, -(R₇ₐ)ₙ-M-R_{7b}, Het¹ und Het², wobei die optionalen Substituenten an allen Aminofunktionen in der obigen Gruppe von Substituenten jeweils unabhängig ausgewählt sind aus R₅ und -A-R₆, und an (ii) einem Stickstoffatom, falls vorhanden, mit Hydroxy oder -A-R₆ substituiert sein kann;
R₇ₐ für gegebenenfalls mit einem oder mehreren Substituenten, ausgewählt aus Halogen, C₁₋₄-Alkylcarbonyl, C₁₋₄-Alkyloxycarbonyl, Aryl, Arylcarbonyl, Aryloxycarbonyl, Het¹ oder Het² substituiertes C₁₋₆-Alkandiyl steht;
R_{7b} für gegebenenfalls mit einem oder mehreren Substituenten, ausgewählt aus Halogen, C₁₋₄-Alkylcarbonyl, C₁₋₄-Alkyloxycarbonyl, Aryl, Arylcarbonyl, Aryloxycarbonyl, Het¹ oder Het² substituiertes C₁₋₆-Alkyl steht;
R₈ für Wasserstoff, C₁₋₆-Alkyl, C₂₋₆-Alkenyl, Aryl-C₁₋₆-alkyl, C₃₋₇-Cycloalkyl, C₃₋₇-Cycloalkyl-C₁₋₆-alkyl, Aryl, Het¹, Het¹-C₁₋₆-Alkyl, Het² oder Het²-C₁₋₆-Alkyl steht;
M -C(=O)-, -O-C(=O)-, -C(=O)-O-, -CH₂-CHOH-, -CHOH-CH₂-, -NR₈-C(=O)-, -(C=O)-NR₈-, -S (=O)₂-, -O-, -S-, -O-S(=O)₂-, -S(=O)₂-O-, -NR₈-S(=O)₂- oder -S(=O)₂-NR₈- bedeutet;
n gleich 0 oder 1 ist;
zur Herstellung eines zur Hemmung der HCV-Aktivität in einem mit HCV infizierten Säuger geeigneten Arzneimittels.

2. Verwendung nach Anspruch 1, wobei es sich bei Q₂ um einen Rest der Formel (III) handelt.

3. Verwendung nach Anspruch 1, wobei es sich bei Q₂ um einen Rest der Formel (IV) handelt.

4. Verwendung nach Anspruch 1, wobei es sich bei Q₂ um einen Rest der Formel (V) handelt.

5. Verwendung nach Anspruch 1, wobei es sich bei Q₂ um einen Rest der Formel (VI) handelt.

6. Verwendung nach Anspruch 1, wobei es sich bei Q₂ um einen Rest der Formel (VII) handelt.

7. Verwendung nach Anspruch 2, wobei A für -C(=O)- oder C₁₋₆-Alkandiyl steht, R₅ für Wasserstoff oder C₁₋₆-Alkyl steht, oder zusammengenommen mit -A-R₆ und mit dem Stickstoffatom, an das es gebunden ist, einen Het¹ bildet; R₆ für C₁₋₆-Alkyloxy, Het¹, Het², Aryl oder Amino steht und, falls -A- nicht C₁₋₆-Alkandiyl ist, auch für C₁₋₆-Alkyl, Het¹-C₁₋₄-Alkyl, Het²-C₁₋₄-Alkyl, Aryl-C₁₋₄-alkyl oder Amino-C₁₋₄-alkyl stehen kann, wobei die Aminogruppen in der Definition von R₆ jeweils gegebenenfalls mit einem oder mehreren Substituenten, ausgewählt aus C₁₋₄-Alkyl, Aryl-C₁₋₄-alkyl, Het¹-C₁₋₄-Alkyl oder Het²-C₁₋₄-Alkyl, substituiert sein können.

8. Verwendung nach Anspruch 3, wobei R¹⁴ und R¹⁵ jeweils entweder beide für Wasserstoff oder für Methyl stehen.

9. Verwendung nach Anspruch 4, wobei R₁₂ für Wasserstoff und R₁₃ für Wasserstoff oder gegebenenfalls mit Aryl substituiertes C₁₋₆-Alkyl steht.

10. Verwendung nach Anspruch 6, wobei Haryl für Thiazolyl oder Oxazolyl steht, die beide gegebenenfalls mit C₁₋₆-Alkyl oder Het²-Amino substituiert sein können.

11. Verwendung nach einem der Ansprüche 1 bis 10, wobei R₂ für Wasserstoff, R₃ für Aryl-C₁₋₄-alkyl und R₄ für C₁₋₆-Alkyl steht.

12. Verwendung nach Anspruch 1, wobei es sich bei der Verbindung um:
{3-[(2-Acetylaminobenzoxazol-6-sulfonyl)isobutylamino]-1-benzyl-2-hydroxypropyl}carbaminsäure-thiazol-5-ylmethylester;
(6-{[2-Hydroxy-4-phenyl-3-(thiazol-5-ylmethoxycarbonylamino)butyl]isobutylsulfamoyl} benzoxazol-2-yl)carbaminsäureethylester;
[1-Benzyl-2-hydroxy-3-({2-[(6-hydroxypyridin-3-carbonyl)amino]benzoxazol-6-sulfonyl}isobutylamino)propyl]carbaminsäure-thiazol-5-ylmethylester;
[1-Benzyl-2-hydroxy-3-(isobutyl-{2-[(pyridin-3-carbonyl)amino]benzoxazol-6-sulfonyl}amino)propyl]carbaminsäure-thiazol-5-ylmethylester;
{1-Benzyl-2-hydroxy-3-[isobutyl-(2-pyrrolidin-1-ylbenzoxazol-6-sulfonyl)amino]propyl}carbaminsäure-thiazol-5-ylmethylester;
[1-Benzyl-2-hydroxy-3-(isobutyl-{2-[methyl-(2-pyrrolidin-1-ylethyl)amino]benzoxazol-6-sulfonyl}amino)propyl]carbaminsäure-thiazol-5-ylmethylester;
[1-Benzyl-2-hydroxy-3-(isobutyl-{2-[2-(4-methylpiperazin-1-yl)acetylamino]benzoxazol-6-sulfonyl}amino)propyl]carbaminsäure-thiazol-5-ylmethylester;
[1-Benzyl-2-hydroxy-3-(isobutyl-{2-[methyl-(5-oxopyrrolidin-2-carbonyl)amino]benzoxazol-6-sulfonyl}amino)propyl]carbaminsäure-thiazol-5-ylmethylester;
[1-Benzyl-2-hydroxy-3-(isobutyl-{2-[methyl-(pyridin-4-carbonyl)amino]benzoxazol-6-sulfonyl}amino)propyl]carbaminsäure-thiazol-5-ylmethylester;
[1-Benzyl-3-({2-[(furan-3-carbonyl)methylamino]benzoxazol-6-sulfonyl}isobutylamino)-2-hydroxypropyl]carbaminsäure-thiazol-5-ylmethylester;
[1-Benzyl-2-hydroxy-3-(isobutyl-{2-[(1-methylpyrrolidin-2-carbonyl)amino]benzoxazol-6-sulfonyl}amino)propyl]carbaminsäure-thiazol-5-ylmethylester;
{1-Benzyl-3-[(3-benzyl-3H-benzimidazol-5-sulfonyl)isobutylamino]-2-hydroxypropyl}carbaminsäure-thiazol-5-ylmethylester;
{3-[(2-Aminobenzthiazol-6-sulfonyl)isobutylamino]-1-benzyl-2-hydroxypropyl}carbaminsäure-thiazol-5-ylmethylester;
(1-Benzyl-3-{[2-(2-dimethylaminoethylamino)benzothiazol-6-sulfonyl]isobutylamino}-2-hydroxypropyl)carbaminsäure-thiazol-5-ylmethylester;
(1-Benzyl-2-hydroxy-3-{isobutyl-[2-(2-pyrrolidin-1-ylethylamino)benzothiazol-6-sulfonyl]amino}propyl)carbaminsäure-thiazol-5-ylmethylester;
(1-Benzyl-2-hydroxy-3-{isobutyl-[2-(2-pyrrolidin-1-ylethylamino)benzothiazol-6-sulfonyl]amino}propyl)carbaminsäure-thiazol-5-ylmethylester, Trifluoracetatsalz;
(1-Benzyl-3-{[2-(3-dimethylaminopropylamino)benzothiazol-6-sulfonyl]-isobutylamino}-2-hydroxypropyl)carbaminsäure-thiazol-5-ylmethylester;
(1-Benzyl-2-hydroxy-3-{isobutyl-[2-(2-piperazin-1-yl-ethylamino)benzothiazol-6-sulfonyl]amino}propyl)carbaminsäure-thiazol-5-ylmethylester;
{3-[(2-Aminobenzoxazol-6-sulfonyl)isobutylamino]-1-benzyl-2-hydroxypropyl}carbaminsäure-thiazol-5-ylmethylester;
{3-[(3H-Benzimidazol-5-sulfonyl)isobutylamino]-1-benzyl-2-hydroxypropyl}carbaminsäure-thiazol-5-ylmethylester;
(3-{[2-(Acetylmethylamino)benzothiazol-6-sulfonyl]isobutylamino}-1-benzyl-2-hydroxypropyl)carbaminsäure-thiazol-5-ylmethylester;
{3-[(2-Aminobenzoxazol-6-sulfonyl)pyridin-2-ylmethylamino]-1-benzyl-2-hydroxypropyl}carbaminsäure-thiazol-5-ylmethylester, Trifluoracetatsalz;
[1-Benzyl-2-hydroxy-3-(isobutyl-{2-[(5-oxopyrrolidin-2-carbonyl)amino]benzoxazol-6-sulfonyl}amino)propyl]carbaminsäure-thiazol-5-ylmethylester
[1-Benzyl-2-hydroxy-3-(isobutyl-{2-[(5-oxopyrrolidin-2-carbonyl)amino]benzoxazol-6-sulfonyl}amino)propyl]carbaminsäure-thiazol-5-ylmethylester;
[1-Benzyl-3-({2-[(furan-3-carbonyl)amino]benzoxazol-6-sulfonyl}isobutylamino)-2-hydroxypropyl]carbaminsäure-thiazol-5-ylmethylester;
[1-Benzyl-2-hydroxy-3-(isobutyl-{2-[(1-methylpiperidin-4-carbonyl)amino]benzoxazol-6-sulfonyl}amino)propyl]carbaminsäure-thiazol-5-ylmethylester;
[1-Benzyl-2-hydroxy-3-(isobutyl-{2-[(pyridin-2-carbonyl)amino]benzoxazol-6-sulfonyl}amino)propyl]carbaminsäure-thiazol-5-ylmethylester;
{3-[(2-Aminobenzoxazol-6-sulfonyl)isobutylamino]-1-benzyl-2-hydroxypropyl}carbaminsäure-2-chlorthiazol-5-ylmethylester;
(1-Benzyl-3-{[2-(2-dimethylaminoacetylamino)benzoxazol-6-sulfonyl]isobutylamino}-2-hydroxypropyl)carbaminsäure-thiazol-5-ylmethylester;
{1-Benzyl-2-hydroxy-3-[isobutyl-(2-piperazin-1-yl-benzoxazol-6-sulfonyl)amino]propyl}carbaminsäure-thiazol-5-ylmethylester;
{1-Benzyl-2-hydroxy-3-[isobutyl-(2-piperidin-1-ylbenzoxazol-6-sulfonyl)amino]propyl}carbaminsäure-thiazol-5-ylmethylester;
{1-Benzyl-2-hydroxy-3-[isobutyl-(2-{2-[methyl-(2-pyrrolidin-1-ylethyl)amino]acetylamino}benzoxazol-6-sulfonyl)amino]propyl}carbaminsäure-thiazol-5-ylmethylester;
{1-Benzyl-3-[(2-dimethylaminobenzoxazol-6-sulfonyl)isobutylamino]-2-hydroxypropyl}carbaminsäure-thiazol-5-ylmethylester;
{3-[(2-Aminobenzoxazol-6-sulfonyl)isobutylamino]-1-benzyl-2-hydroxypropyl}carbaminsäure-oxazol-5-ylmethylester;
[1-Benzyl-2-hydroxy-3-(isobutyl-{2-[(pyridin-4-carbonyl)amino]benzoxazol-6-sulfonyl}amino)propyl]carbaminsäure-thiazol-5-ylmethylester;
[1-Benzyl-2-hydroxy-3-(isobutyl-{2-[methyl(pyridin-2-carbonyl)amino]benzoxazol-6-sulfonyl}amino)propyl]carbaminsäure-thiazol-5-ylmethylester;
[1-Benzyl-2-hydroxy-3-(isobutyl-{2-[methyl-(1-methylpiperidin-3-carbonyl)amino]benzoxazol-6-sulfonyl}amino)propyl]carbaminsäure-thiazol-5-ylmethylester;
[1-Benzyl-2-hydroxy-3-(isobutyl-{2-[methyl-(1-methylpiperidin-4-carbonyl)amino]benzoxazol-6-sulfonyl}amino)propyl]carbaminsäure-thiazol-5-ylmethylester;
[1-Benzyl-3-({2-[(2-chlorpyridin-4-carbonyl)methylamino]benzoxazol-6-sulfonyl}isobutylamino)-2-hydroxypropyl]carbaminsäure-thiazol-5-ylmethylester;
[1-Benzyl-2-hydroxy-3-(isobutyl-{2-[methyl-(1-methylpyrrolidin-2-carbonyl)amino]benzoxazol-6-sulfonyl}amino)propyl]carbaminsäure-thiazol-5-ylmethylester, Trifluoracetatsalz;
{1-Benzyl-2-hydroxy-3-[isobutyl-(3-phenethyl-3H-benzimidazol-5-sulfonyl)amino]propyl}carbaminsäure-thiazol-5-ylmethylester;
{1-Benzyl-2-hydroxy-3-[isobutyl-(3-isobutyl-3H-benzimidazol-5-sulfonyl)amino]propyl}carbaminsäure-thiazol-5-ylmethylester;
[1-Benzyl-2-hydroxy-3-(isobutyl-{4-[2-(pyridin-4-ylamino)thiazol-4-yl]benzolsulfonyl}amino)propyl]carbaminsäure-thiazol-5-ylmethylester;
(1-Benzyl-2-hydroxy-3-{isobutyl-[4-(2-methyloxazol-4-yl)benzolsulfonyl]amino}propyl)carbaminsäure-thiazol-5-ylmethylester; oder
{3-[(2-Aminobenzoxazol-6-sulfonyl)isobutylamino]-1-benzyl-2-hydroxypropyl}carbaminsäure-thiazol-5-ylmethylester;
oder um ein *N*-Oxid, Salz oder eine stereoisomere Form davon handelt.

13. Verwendung nach einem der Ansprüche 1 bis 12, wobei der Säuger mit HIV und HCV koinfiziert ist.

14. Verwendung eines Sulfonamids mit der in einem der Ansprüche 1 bis 12 angegebenen Bedeutung bei der Herstellung einer auf die Behandlung oder Bekämpfung einer HCV-Infektion gerichteten pharmazeutischen Zusammensetzung.

15. Kombination eines Sulfonamids mit der in einem der Ansprüche 1 bis 12 angegebenen Bedeutung mit einem weiteren Anti-HCV-Mittel.

16. Kombination nach Anspruch 15, ferner ein Anti-HIV-Mittel umfassend.

## Revendications

1. Utilisation de dérivés de sulfonamides ayant la formule générale (I) 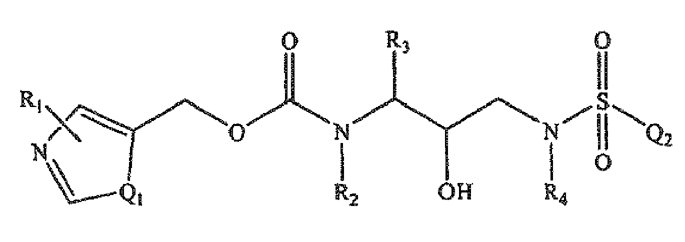 ou d'un N-oxyde, d'un sel, d'une forme stéréoisomère, d'un mélange racémique, d'une prodrogue ou d'esters de ceux-ci, dans laquelle :
Q₁ est -S- ou -O- ;
R₁ est hydrogène, C₁₋₆alkyle, hydroxy, amino, halogène, aminoC₁₋₄alkyle et mono- ou di(C₁₋₄alkyl)amino ;
R₂, R₁₄ et R₁₅ sont, chacun indépendamment, hydrogène ou C₁₋₆alkyle ;
R₃ est C₁₋₆alkyle, aryle, C₃₋₇cycloalkyle, C₃₋₇cycloalkylC₁₋₄alkyle ou arylC₁₋₄alkyle ;
R₄ est hydrogène, C₁₋₄alkyloxycarbonyle, carboxy, aminocarbonyle éventuellement mono- ou di-substitué, mono- ou di (C₁₋₆alkyl)aminocarbonyle, C₃₋₇cycloalkyle, C₂₋₆alcényle, C₂₋₆alcynyle ou C₁₋₆alkyle éventuellement substitué par un ou plusieurs substituants choisis chacun indépendamment parmi aryle, Het¹, Het², C₃₋₇cycloalkyle, C₁₋₄alkyloxycarbonyle, carboxy, aminocarbonyle, mono- ou di(C₁₋₄alkyl)aminocarbonyle, aminosulfonyle, C₁₋₄alkylS(=O)ₜ, hydroxy, cyano, halogène ou amino éventuellement mono- ou disubstitué, où les substituants sont choisis chacun indépendamment parmi C₁₋₄alkyle, aryle, arylC₁₋₄alkyle, C₃₋₇cycloalkyle, C₃₋₇cycloalkylC₁₋₄alkyle, Het¹, Het², Het¹C₁₋₄alkyle et Het²C₁₋₄alkyle ;
Q₂ est un radical de formule (III), (IV), (V), (VI) ou (VII) 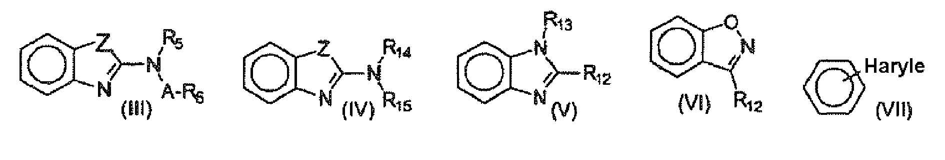 et est à relier au reste de la molécule par l'intermédiaire d'un atome de carbone quelconque du cycle phényle ou phényle condensé ;
Z est O ou S ;
A est C₁₋₆alcanediyle, -C(=O)-, -C(=S)-, -S (=O)₂-, C₁₋₆alcanediyl-C(=O)-, C₁₋₆alcanediyl-C(=S)- ou C₁₋₆alcanediyl-S(=O)₂- ; où le point de liaison à l'atome d'azote est le groupement C₁₋₆alcanediyle dans les motifs contenant ledit groupement ;
R₅ est hydrogène, hydroxy, C₁₋₆alkyle, Het¹C₁₋₆alkyle, Het²C₁₋₆alkyle ou aminoC₁₋₆alkyle, où le groupement amino peut éventuellement être mono- ou disubstitué par C₁₋₄alkyle ;
R₆ est C₁₋₆alkyloxy, Het¹, Het¹oxy, Het², Het²oxy, aryle, aryloxy ou amino ; et dans le cas où -A- est autre que C₁₋₆alcanediyle, alors R₆ peut également être C₁₋₆alkyle, Het¹C₁₋₄alkyle, Het¹oxyC₁₋₄alkyle, Het²C₁₋₄alkyle, Het²oxy-C₁₋₄alkyle, arylC₁₋₄alkyle, aryloxyC₁₋₄alkyle ou aminoC₁₋₄alkyle ; où chacun des groupements amino dans la définition de R₆ peut éventuellement être substitué par un ou plusieurs substituants choisis parmi C₁₋₄-alkyle, C₁₋₄alkylcarbonyle, C₁₋₄alkyloxycarbonyle, aryle, arylcarbonyle, aryloxycarbonyle, Het¹, Het², arylC₁₋₄alkyle, Het¹C₁₋₄alkyle et Het²C₁₋₄alkyle ; et
R₅ et -A-R₆, pris conjointement avec l'atome d'azote auquel ils sont liés, peuvent également former Het¹ ou Het² ;
R₁₂ est hydrogène, -NH₂, -N(R₅)(AR₆), -C₁₋₆alkyle ou C₁₋₆alkyl-W-R₁₇, ou chaque C₁₋₆alkyle peut éventuellement être substitué par halogène, hydroxy, aryle, Het¹, Het², amino, ou mono- ou di(C₁₋₄alkyl)amino ;
W est oxy, carbonyle, oxycarbonyle, carbonyloxy, oxycarbonyloxy, amino, aminocarbonyle, carbonylamino ou soufre ;
R₁₃ est hydrogène ou C₁₋₆alkyle éventuellement substitué par un substituant choisi dans le groupe constitué de aryle, Het¹, Het², hydroxy, halogène ou amino, où le groupement amino peut éventuellement être mono- ou di-substitué par C₁₋₄alkyle ;
R₁₇ est C₁₋₆alkyle, aryle, Het¹ ou Het² ;
Haryle est un hétérocycle monocyclique, bicyclique ou tricyclique, aromatique, ayant de 3 à 14 membres du cycle qui contient un ou plusieurs membres hétéroatomes du cycle choisis parmi azote, oxygène et soufre, et qui peut éventuellement être substitué sur (i) un ou plusieurs atomes de carbone par un substituant choisi dans le groupe constitué de C₁₋₆alkyle, halogène, hydroxy, amino éventuellement mono- ou disubstitué, nitro, cyano, halogénoC₁₋₆alkyle, carboxy, C₃₋₇cycloalkyle, aminocarbonyle éventuellement mono- ou di-substitué, méthylthio, méthylsulfonyle, aryle, -(R₇ₐ)ₙ-M-R_{7b}, Het¹ et Het² ; où les substituants éventuels, sur toute fonction amino dans le groupe de substituants ci-dessus, sont indépendamment choisis parmi R₅ et -A-R₆ ; et sur (ii) un atome d'azote, s'il en existe, par hydroxy ou -A-R₆ ;
R₇ₐ est C₁₋₆alcanediyle éventuellement substitué par un ou plusieurs substituants choisis parmi halogène, C₁₋₄alkylcarbonyle, C₁₋₄alkyloxycarbonyle, aryle, arylcarbonyle, aryloxycarbonyle, Het¹ ou Het² ;
R_{7b} est C₁₋₆alkyle éventuellement substitué par un ou plusieurs substituants choisis parmi halogène, C₁₋₄alkylcarbonyle, C₁₋₄alkyloxycarbonyle, aryle, arylcarbonyle, aryloxycarbonyle, Het¹ ou Het² ;
R₈ est hydrogène, C₁₋₆alkyle, C₂₋₆alcényle, arylC₁₋₆alkyle, C₃₋₇cycloalkyle, C₃₋₇cycloalkyl-C₁₋₆alkyle, aryle, Het¹, Het¹C₁₋₆alkyle, Het² ou Het²C₁₋₆alkyle ;
M est défini par -C(=O)-, -O-C(=O)-, -C(=O)-O-, -CH₂-CHOH-, -CHOH-CH₂-, -NR₈-C(=O)-, - (C=O) -NR₈-, -S (=O)₂-, -O-, -S-, -O-S (=O)₂-, -S (=O)₂-O-, -NR₈-S(=O)₂ ou -S(=O)₂-NR₈- ;
n est zéro ou 1 ;
dans la fabrication d'un médicament utile pour inhiber l'activité du VHC chez un mammifère infecté par le VHC.

2. Utilisation selon la revendication 1, **caractérisée en ce que** Q₂ est un radical de formule (III).

3. Utilisation selon la revendication 1, **caractérisée en ce que** Q₂ est un radical de formule (IV).

4. Utilisation selon la revendication 1, **caractérisée en ce que** Q₂ est un radical de formule (V).

5. Utilisation selon la revendication 1, **caractérisée en ce que** Q₂ est un radical de formule (VI).

6. Utilisation selon la revendication 1, **caractérisée en ce que** Q₂ est un radical de formule (VII).

7. Utilisation selon la revendication 2, **caractérisée en ce que** A est -C(=O)- ou C₁₋₆alcanediyle, R₅ est hydrogène ou C₁₋₆alkyle ; ou, pris conjointement avec -A-R₆ et avec l'atome d'azote auquel il est lié, forme Het¹ ; R₆ est C₁₋₆alkyloxy, Het¹, Het², aryle ou amino ; et dans le cas où -A- est autre que C₁₋₆alcanediyle, alors R₆ peut également être C₁₋₆alkyle, Het¹C₁₋₄alkyle, Het²C₁₋₄alkyle, aryl-C₁₋₄alkyle ou aminoC₁₋₄alkyle ; où chacun des groupements amino dans la définition de R₆ peut éventuellement être substitué par un ou plusieurs substituants choisis parmi C₁₋₄alkyle, aryl-C₁₋₄alkyle, Het¹C₁₋₄alkyle ou Het²C₁₋₄alkyle.

8. Utilisation selon la revendication 3, **caractérisée en ce que** R¹⁴ et R¹⁵ sont tous deux hydrogène ou sont tous deux méthyle.

9. Utilisation selon la revendication 4, **caractérisée en ce que** R₁₂ est hydrogène et R₁₃ est hydrogène ou C₁₋₆alkyle éventuellement substitué par aryle.

10. Utilisation selon la revendication 6, **caractérisée en ce que** Haryle est thiazolyle ou oxazolyle, qui peuvent tous deux être éventuellement substitués par C₁₋₆alkyle ou Het²amino.

11. Utilisation selon l'une quelconque des revendications 1 à 10, **caractérisée en ce que** R₂ est hydrogène, R₃ est arylC₁₋₄alkyle et R₄ est C₁₋₆alkyle.

12. Utilisation selon la revendication 1, **caractérisée en ce que** le composé est :
l'ester de thiazol-5-ylméthyle d'acide {3-[(2-acétylaminobenzooxazol-6-sulfonyl)-isobutyl-amino]-1-benzyl-2-hydroxypropyl}-carbamique ;
l'ester éthylique d'acide (6-{[2-hydroxy-4-phényl-3-(thiazol-5-ylméthoxycarbonylamino)-butyl]-isobutylsulfamoyl}-benzooxazol-2-yl)-carbamique ;
l'ester de thiazol-5-ylméthyle d'acide [1-benzyl-2-hydroxy-3-({2-[(6-hydroxypyridine-3-carbonyl)-amino]-benzooxazole-6-sulfonyl}-isobutylamino)-propyl]-carbamique ;
l'ester de thiazol-5-ylméthyle d'acide [1-benzyl-2-hydroxy-3-(isobutyl-{2-[(pyridine-3-carbonyl)-amino]-benzooxazol-6-sulfonyl}-amino)-propyl]-carbamique ;
l'ester de thiazol-5-ylméthyle d'acide {1-benzyl-2-hydroxy-3-[isobutyl-(2-pyrrolidin-1-ylbenzooxazol-6-sulfonyl)-amino]-propyl}-carbamique ;
l'ester de thiazol-5-ylméthyle d'acide [1-benzyl-2-hydroxy-3-(isobutyl-{2-[méthyl-(2-pyrrolidin-1-yléthyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[2-(4-méthylpipérazin-1-yl)-acétylamino]-benzooxazol-6-sulfonyl}-amino)-propyl]-carbamique ;
l'ester de thiazol-5-ylméthyle d'acide [1-benzyl-2-hydroxy-3-(isobutyl-{2-[méthyl-(5-oxopyrrolidine-2-carbonyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[méthyl-(pyridine-4-carbonyl)-amino]-benzooxazol-6-sulfonyl}-amino)-propyl]-carbamique ;
l'ester de thiazol-5-ylméthyle d'acide [1-benzyl-3-({2-[(furan-3-carbonyl)-méthylamino]-benzooxazol-6-sulfonyl}-isobutylamino)-2-hydroxypropyl]-carbamique ;
l'ester de thiazol-5-ylméthyle d'acide [1-benzyl-2-hydroxy-3-(isobutyl-{2-[(1-méthylpyrrolidine-2-carbonyl)-amino]-benzooxazol-6-sulfonyl}-amino)-propyl]-carbamique ;
l'ester de thiazol-5-ylméthyle d'acide {1-benzyl-3-[(3-benzyl-3H-benzoimidazol-5-sulfonyl)-isobutylamino]-2-hydroxypropyl}-carbamique ;
l'ester de thiazol-5-ylméthyle d'acide {3-[(2-aminobenzothiazol-6-sulfonyl)-isobutylamino]-1-benzyl-2-hydroxypropyl}-carbamique ;
l'ester de thiazol-5-ylméthyle d'acide (1-benzyl-3-{[2-(2-diméthylaminoéthylamino)-benzothiazol-6-sulfonyl]-isobutylamino}-2-hydroxypropyl)-carbamique ;
l'ester de thiazol-5-ylméthyle d'acide (1-benzyl-2-hydroxy-3-{isobutyl-[2-(2-pyrrolidin-1-yléthylamino)-benzothiazol-6-sulfonyl]-amino}-propyl)-carbamique ;
le sel de trifluoroacétate d'ester de thiazol-5-ylméthyle d'acide (1-benzyl-2-hydroxy-3-{isobutyl-[2-(2-pyrrolidin-1-yléthylamino)-benzothiazol-6-sulfonyl]-amino}-propyl)-carbamique ;
l'ester de thiazol-5-ylméthyle d'acide (1-benzyl-3-{[2-(3-diméthylaminopropylamino)-benzothiazol-6-sulfonyl]-isobutylamino}-2-hydroxypropyl)-carbamique ;
l'ester de thiazol-5-ylméthyle d'acide (1-benzyl-2-hydroxy-3-{isobutyl-[2-(2-pipérazin-1-yléthylamino)-benzothiazol-6-sulfonyl]-amino}-propyl)-carbamique ;
l'ester de thiazol-5-ylméthyle d'acide {3-[(2-aminobenzooxazol-6-sulfonyl)-isobutylamino]-1-benzyl-2-hydroxypropyl}-carbamique ;
l'ester de thiazol-5-ylméthyle d'acide {3-[(3H-benzoimidazol-5-sulfonyl)-isobutylamino]-1-benzyl-2-hydroxypropyl}-carbamique ;
l'ester de thiazol-5-ylméthyle d'acide (3-{(2-(acétylméthylamino)-benzothiazol-6-sulfonyl]-isobutylamino}-1-benzyl-2-hydroxypropyl)-carbamique ;
le sel de trifluoroacétate d'ester de thiazol-5-ylméthyle d'acide {3-[(2-aminobenzooxazol-6-sulfonyl)-pyridin-2-ylméthylamino]-1-benzyl-2-hydroxypropyl}-carbamique ;
l'ester de thiazol-5-ylméthyle d'acide [1-benzyl-2-hydroxy-3-(isobutyl-{2-[(5-oxopyrrolidine-2-carbonyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[(5-oxopyrrolidine-2-carbonyl)-amino]-benzooxazol-6-sulfonyl}-amino)-propyl]-carbamique ;
l'ester de thiazol-5-ylméthyle d'acide [1-benzyl-3-({2-[(furane-3-carbonyl)-amino]-benzooxazol-6-sulfonyl}-isobutylamino)-2-hydroxypropyl]-carbamique ;
l'ester de thiazol-5-ylméthyle d'acide [1-benzyl-2-hydroxy-3-(isobutyl-{2-[(1-méthylpipéridine-4-carbonyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[(pyridine-2-carbonyl)-amino]-benzooxazol-6-sulfonyl}-amino)-propyl]-carbamique ;
l'ester de 2-chlorothiazol-5-ylméthyle d'acide {3-[(2-aminobenzooxazol-6-sulfonyl)-isobutylamino]-1-benzyl-2-hydroxypropyl}-carbamique ;
l'ester de thiazol-5-ylméthyle d'acide (1-benzyl-3-{[2-(2-diméthylaminoacétylamino)-benzooxazol-6-sulfonyl]-isobutylamino}-2-hydroxypropyl)-carbamique ;
l'ester de thiazol-5-ylméthyle d'acide {1-benzyl-2-hydroxy-3-[isobutyl-(2-pipérazin-1-yl-benzooxazol-6-sulfonyl)-amino]-propyl}-carbamique ;
l'ester de thiazol-5-ylméthyle d'acide {1-benzyl-2-hydroxy-3-[isobutyl-(2-pipéridin-1-yl-benzooxazol-6-sulfonyl)-amino]-propyl}-carbamique ;
l'ester de thiazol-5-ylméthyle d'acide {1-benzyl-2-hydroxy-3-[isobutyl-(2-{2-[méthyl-(2-pyrrolidin-1-yléthyl)-amino]-acétylamino}-benzooxazol-6-sulfonyl)-amino]-propyl}-carbamique ;
l'ester de thiazol-5-ylméthyle d'acide {1-benzyl-3-[(2-diméthylaminobenzooxazol-6-sulfonyl)-isobutylamino]-2-hydroxypropyl}-carbamique ;
l'ester d'oxazol-5-ylméthyle d'acide {3-[(2-aminobenzooxazol-6-sulfonyl)-isobutylamino]-1-benzyl-2-hydroxypropyl}-carbamique ;
l'ester de thiazol-5-ylméthyle d'acide [1-benzyl-2-hydroxy-3-(isobutyl-{2-[(pyridine-4-carbonyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[méthyl-(pyridine-2-carbonyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[méthyl-(1-méthylpipéridine-3-carbonyl)-amino]-benzooxazol-6-sulfonyl}-amino)-propyl]-carbamique ;
l'ester de thiazol-5-ylméthyle d'acide [1-benzyl-2-hydroxy-3-(isobutyl-{2-[méthyl-(1-méthylpipéridine-4-carbonyl)-amino]-benzooxazol-6-sulfonyl}-amino)-propyl]-carbamique ;
l'ester de thiazol-5-ylméthyle d'acide [1-benzyl-3-({2-[(2-chloropyridine-4-carbonyl)-méthylamino]-benzooxazol-6-sulfonyl}-isobutylamino)-2-hydroxypropyl]-carbamique ;
le sel de trifluoroacétate d'ester de thiazol-5-ylméthyle d'acide [1-benzyl-2-hydroxy-3-(isobutyl-{2-[méthyl-(1-méthylpyrrolidine-2-carbonyl)-amino]-benzooxazol-6-sulfonyl}-amino)-propyl]-carbamique ;
l'ester de thiazol-5-ylméthyle d'acide {1-benzyl-2-hydroxy-3-[isobutyl-(3-phénéthyl-3H-benzoimidazol-5-sulfonyl)-amino]-propyl}-carbamique ;
l'ester de thiazol-5-ylméthyle d'acide {1-benzyl-2-hydroxy-3-[isobutyl-(3-isobutyl-3H-benzoimidazol-5-sulfonyl)-amino]-propyl}-carbamique ;
l'ester de thiazol-5-ylméthyle d'acide [1-benzyl-2-hydroxy-3-(isobutyl-{4-[2-(pyridin-4-ylamino)-thiazol-4-yl]-benzènesulfonyl}-amino)-propyl]-carbamique ;
l'ester de thiazol-5-ylméthyle d'acide (1-benzyl-2-hydroxy-3-{isobutyl-[4-(2-méthyloxazol-4-yl)-benzènesulfonyl]-amino}-propyl)-carbamique ou
l'ester de thiazol-5-ylméthyle d'acide {3-[(2-aminobenzooxazol-6-sulfonyl)-isobutylamino]-1-benzyl-2-hydroxypropyl}-carbamique ;
ou un N-oxyde, un sel, une forme stéréoisomère de ceux-ci.

13. Utilisation selon l'une quelconque des revendications 1 à 12, **caractérisée en ce que** le mammifère est infecté à la fois par le VIH et par le VHC.

14. Utilisation d'un sulfonamide, tel que défini dans l'une quelconque des revendications 1 à 12, dans la fabrication d'une composition pharmaceutique destinée à traiter ou à lutter contre l'infection par le VHC.

15. Combinaison d'un sulfonamide, tel que défini dans l'une quelconque des revendications 1 à 12, avec un autre agent anti-VHC.

16. Combinaison selon la revendication 15 comprenant en outre un agent anti-VIH.
